(19) Europäisches Patentamt
European Patent Office
Office européen des brevets

(11) EP 1 729 754 B1

(12) EUROPEAN PATENT SPECIFICATION

(45) Date of publication and mention of the grant of the patent:
**02.07.2008 Bulletin 2008/27**

(21) Application number: **04811076.1**

(22) Date of filing: **01.12.2004**

(51) Int Cl.:
***A61K 31/5375*** (2006.01) ***A61K 31/5377*** (2006.01)
***A61P 15/12*** (2006.01)

(86) International application number:
**PCT/US2004/038221**

(87) International publication number:
**WO 2005/060949 (07.07.2005 Gazette 2005/27)**

(54) **SELECTIVE NOREPINEPHRINE REUPTAKE INHIBITORS FOR THE TREATMENT OF HOT FLASHES**

SELEKTIVE NOREPINEPHRIN-WIEDERAUFNAHMEHEMMER ZUR BEHANDLUNG VON HITZEWALLUNGEN

INHIBITEURS SELECTIFS DU RECAPTAGE DE LA NOREPINEPHRINE POUR TRAITER LES BOUFFEES DE CHALEUR

(84) Designated Contracting States:
**AT BE BG CH CY CZ DE DK EE ES FI FR GB GR HU IE IS IT LI LT LU MC NL PL PT RO SE SI SK TR**

(30) Priority: **12.12.2003 US 529428 P**

(43) Date of publication of application:
**13.12.2006 Bulletin 2006/50**

(60) Divisional application:
**08151775.7**

(73) Proprietor: **ELI LILLY AND COMPANY**
**Indianapolis, Indiana 46285 (US)**

(72) Inventors:
- **ALLEN, Albert, John**
  **Indianapolis, Indiana 46228 (US)**
- **HEMRICK-LUECKE, Susan**
  **Plainfield, Indiana 46168 (US)**
- **SUMNER, Calvin, Russell**
  **Lebanon, Indiana 46052 (US)**
- **WALLACE, Owen, Brendan**
  **Zionsville, Indiana 46077 (US)**

(74) Representative: **Hiscock, Ian James et al**
**European Patent Operations,**
**Eli Lilly and Company Limited,**
**Lilly Research Centre,**
**Erl Wood Manor,**
**Sunninghill Road Windlesham GU20 6PH (GB)**

(56) References cited:
**EP-A- 0 534 756**
**WO-A-02/40006**
**WO-A-02/078691**
**WO-A-03/037334**
**WO-A-20/04017977**
**WO-A-20/04018441**
**WO-A-20/04035036**
**WO-A-20/04035058**
**WO-A-20/04052858**
**WO-A-20/05023802**
**WO-A-20/05047272**

- **LOPRINZI C L ET AL: "Venlafaxine in management of hot flashes in survivors of breast cancer: a randomised controlled trial" LANCET, vol. 356, no. 9247, 16 December 2000 (2000-12-16), pages 2059-2063, XP004264310 ISSN: 0140-6736**
- **QUELLA S K ET AL: "PILOT EVALUATION OF VENLAFAXINE FOR THE TREATMENT OF HOT FLASHES IN MEN UNDERGOING ANDROGEN ABLATION THERAPY FOR PROSTATE CANCER" JOURNAL OF UROLOGY, BALTIMORE, MD, US, vol. 162, no. 1, July 1999 (1999-07), pages 98-102, XP009026289 ISSN: 0022-5347**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 729 754 B1

## Description

### Background of the Invention

### Field of the Invention

The present invention relates to the fields of pharmaceutical chemistry and central nervous system medicine. More particularly, the present invention provides medicaments for the prevention or treatment of hot flashes or vasomotor symptoms.

### Description of Related Art

#### Hot Flashes

Hot flashes (also known as "hot flushes") are characterized by a warming sensation that begins in the chest and moves towards the neck and head, and are often accompanied by sweating, palpitations, and cutaneous flushing. The episodes last from 30 seconds to 10 minutes. The majority of postmenopausal women will experience hot flashes and night sweats (vasomotor symptoms), with a significant percentage of these women continuing to suffer symptoms for more than five years (Psychosom. Med. (1965) 27:266; Med. Gynecol. Soc. (1969) 4: 268). Women who have undergone bilateral oophorectomy, radiotherapy, or treatment with GnRH (gonadotropin releasing hormone) agonists are particularly prone to hot flushes (Br. J. Obstet. Gynaecol. (1977) 84:769). Men have also been reported to experience vasomotor symptoms following treatment with a GnRH agonist (N. Engl. J. Med. (1981) 305:663) or after orchidectomy (Urology (1980) 16:620).

In spite of being identified as an ailment of menopause for hundreds of years, the precise mechanism underlying the cause of hot flashes is not clear. However, the link with declining estrogen levels (due to natural menopause or otherwise) is widely accepted. As mentioned above, women undergoing natural, surgical, or chemically-induced ovarian failure suffer from vasomotor symptoms. In addition, the anti-cancer drug tamoxifen is known to induce hot flushes in more than 50% of patients (Arch. Intern. Med. (1991) 151:1842), presumably due to antagonism (or partial agonism) of the estrogen receptor in the hypothalamus. Interestingly, women with low estrogen levels due to ovarian dysgenesis do not suffer from hot flashes unless they are first given hormone replacement therapy and then have it discontinued (Clin. Endocrinol. (Oxf) (1985) 22:293). Estrogen or hormone replacement therapy (ERT or HRT, respectively) is current the gold standard treatment and is effective in greater than 80% of women who initiate treatment, which again is supportive of an estrogenic role in the etiology of hot flushes.

The hot flash event itself is thought to be centrally mediated, resulting from a transient lowering of the thermoregulatory set point in the hypothalamus (for a review, see: Can. J. Physiol. Pharmacol. (1987) 65:1312). Regulation of the thermoregulatory process may involve catecholamines, estrogen, testosterone, opioids, and serotonin, among others (for a review, see: Mayo. Clin. Proc. (2002) 77:1207). In fact, compounds that modulate the signaling pathway of each of these hormones/neurotransmitters have been evaluated for the treatment of hot flushes. Clonidine, an α-adrenergic agonist used to treat hypertension, has been used clinically with mixed results. Several studies have reported some efficacy (e.g., Ann. Intern. Med. (2000) 132:788; Br. Med. J. (1974) i, 409), while others were unable to confirm, effectiveness (*Maturitas* (1978) 1:21; Med. J. Aust. (1986) 144:369). As mentioned above, estrogen is highly efficacious, and is the current treatment of choice where not contraindicated. Danazol, a corticosteroid with anti-androgen properties, showed some efficacy in treating hot flashes (Fertil. Steril. (1985) 43:401) and a combination of methyltestosterone with estrogen showed significant symptom relief. There have been conflicting reports surrounding the ability of the opioid receptor antagonist naloxone to effectively treat hot flushes (Br. J. Obstet. Gynaecol. (1981) 88:919; J. Clin. Endocrinol. Metab. (1984) 58:578; Clin. Endocrinol. (1985) 22:293). Accounts of clinical efficacy with anti-depressants for treating hot flushes have been described recently (for a review of the role of serotonin in hot flashes, see *Maturitas* (2000) 36: 155). For example, the selective serotonin reuptake inhibitors (SSRIs) fluoxetine (J. Clin. Oncol. (2002) 20:1583) and paroxetine (JAMA (2003) 289: 2827) have demonstrated some efficacy. Furthermore, venlafaxine, which has mixed serotonin and norepinephrine reuptake inhibition properties, has recently been shown to be clinically efficacious (Lancet (2000) 356:2059).

In addition to the agents describe above that have been postulated to affect the themoregulatory set point described above, several other hot flash treatments have been investigated. Of these, the progestins have demonstrated noteworthy efficacy. For example, megestrol has been shown to be equally efficacious in men and women suffering from hot flashes (N. Engl. J. Med. (1994) 331:347). Depomedroxyprogesterone acetate has also been investigated (Obstet. Gynecol. (1984) 63:1) as has transdermal progesterone cream (Obstet. Gynecol. (1999) 94:225). In addition, combinations of estrogen and progestins have demonstrated efficacy and are widely used by post-menopausal women who have not undergone hysterectomy. Other therapies explored for hot flash treatment include tibolone, a compound

with estrogenic, androgenic, and progestogenic activity (Br. J. Obstet. Gynecol. (1998) 105:904), the anti-epileptic gabapentin (Neurology (2000) 54:2161), the dopamine antagonist veralipride (Obstet. Gynecol. (1998) 72:688), vitamins (J. Clin. Oncol. (1998) 16:495), herbal remedies such as black cohosh (J. Clin. Oncol. (2001) 19:2739), and soy proteins (J. Nutr. (2001) 131 (11, suppl.): 3095s).

More recently, International (PCT) publication No. WO 03/037334 (8 May 2003) has proposed the use of a tachykinin receptor antagonist, and especially a neurokinin-1 (NK-1) receptor antagonist, for the manufacture of a medicament for the treatment of hot flashes.

In spite of the apparent large number of treatments for vasomotor symptoms, all the current therapies either suffer from poor efficacy, are associated with unacceptable side effects, or are contraindicated for certain patient populations. For example, estrogen replacement therapy is not recommended for women with a history of breast cancer, uterine cancer, ovarian cancer, or venous thromboembolism. Recent data also suggest that HRT may not be suitable for women with coronary artery disease. Generally, non-hormonal treatments are not fully efficacious (e.g., clonidine) and/or cause adverse effects (venlafaxine, gabapentin). There therefore remains an unmet medical need for hot flash therapies that overcome the liabilities of current treatments.

## Summary of the Invention

The present invention provides the use of a selective norepinephrine reuptake inhibitor as defined hereinafter for the manufacture of a medicament for the treatment or prevention of hot flashes or vasomotor symptoms.

Further scope of the applicability of the present invention will become apparent from the detailed description provided below. However, it should be understood that the detailed description and specific examples, while indicating preferred embodiments of the present invention, are given by way of illustration only since various changes and modifications within the spirit and scope of the invention will become apparent to those skilled in the art from this detailed description.

## Detailed Description of the Invention

The following detailed description of the invention is provided to aid those skilled in the art in practicing the present invention.

The present invention provides the use of a norepinephrine reuptake inhibitor as defined hereinafter for the manufacture of a medicament for treating or preventing hot flashes or vasomotor symptoms. In a preferred embodiment the patient is a postmenopausal woman. In another preferred embodiment the patient is a woman who has undergone bilateral oophorectomy, radiotherapy, or treatment with a GnRH (gonadotropin releasing hormone) agonist. In another preferred embodiment the patient is a woman who has undergone natural, surgical, or chemically-induced ovarian failure. In another preferred embodiment the patient is a man who has undergone treatment with a GnRH agonist or an orchidectomy. In another preferred embodiment the patient is a person, especially a woman, who has undergone treatment with the anti-cancer drug tamoxifen.

The uses of the present invention rely on a novel mechanism of action, i.e., selective inhibition of norepinephrine reuptake, and comprise administering to a mammal in need of such prophylactic or therapeutic treatment an effective amount of a selective norepinephrine reuptake inhibitor. This mechanism is operative in mammals, with the preferred mammal being a human.

### Norepinephrine Reuptake Inhibitors Useful in the Present Invention

Many compounds, including those discussed at length below, are selective norepinephrine reuptake inhibitors, and no doubt many more will be identified in the future. In the practice of the present invention, it is intended to include reuptake inhibitors which show 50% effective concentrations of about 1000 nM or less, in the protocol described by Wong et al., Drug Development Research, 6, 397 (1985). The norepinephrine reuptake inhibitors useful for the medicaments of the present invention are characterized in being selective for the inhibition of neurotransmitter reuptake relative to their ability to act as direct agonists or antagonists at other receptors. It is preferred that the compounds useful for the medicaments of the present invention are selective for the inhibition of norepinephrine reuptake relative to direct agonist or antagonist activity at other receptors by a factor of at least ten. Preferably, compounds useful for the medicaments of the present invention are selective for the inhibition of norepinephrine reuptake relative to direct agonist or antagonist activity at other receptors by a factor of at least one hundred. In particular it is preferred that the compounds useful for the medicaments of the present invention demonstrate such selective inhibition of norepinephrine reuptake relative to the inhibition of dopamine and serotonin reuptake.

Norepinephrine reuptake inhibitors useful in the compositions and medicaments of the present invention are compounds of formula (IH)

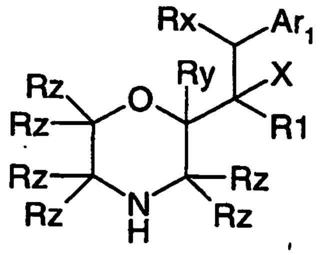

(IH)

wherein,

X is OH, C1-C4 alkoxy, $NH_2$ or NH(C1-C4 alkyl);
Rx is H or C1-C4 alkyl;
Ry is H or C1-C4 alkyl;
each Rz group is independently H or C1-C4 alkyl, with the proviso that not more than 3 Rz groups may be C1-C4 alkyl;
R1 is C1-C6 alkyl (optionally substituted with 1, 2 or 3 halogen atoms and/or with 1 substituent selected from C1-C4 alkylthio (optionally substituted with 1, 2 or 3 fluorine atoms), C1-C4 alkoxy (optionally substituted with 1, 2 or 3 fluorine atoms), C3-C6 cycloalkoxy, C1-C4 alkylsulfonyl, cyano, -CO-O(C1-C2 alkyl), -O-CO-(C1-C2 alkyl) and hydroxy); C2-C6 alkenyl (optionally substituted with 1, 2 or 3 halogen atoms); C3-C6 cycloalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C, S-C or C=C bond; C4-C7 cycloalkylalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C, S-C or C=C bond; or $CH_2Ar2$; and Ar1 and Ar2 are each independently a phenyl ring or a 5- or 6-membered heteroaryl ring each of which is optionally substituted with 1, 2 or 3 substituents (depending upon the number of available substitution positions) each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), -CO-O(C1-C4 alkyl), cyano, -NRR, -CONRR, halo and hydroxy and/or with 1 substituent selected from pyridyl, thiophenyl, phenyl, benzyl and phenoxy each of which is optionally ring-substituted with 1, 2 or 3 substituents each independently selected from halogen, C1-C4 alkyl (optionally substituted with 1, 2 or 3 halogen atoms), C1-C4 alkoxy (optionally substituted with 1, 2 or 3 halogen atoms), carboxy, nitro, hydroxy, cyano, -NRR, -CONRR, $SO_2NRR$ and $SO_2R$); and
each R is independently H or C1-C4 alkyl;

or a pharmaceutically acceptable salt thereof.

With respect to compounds of formula (IH), the term "C1-C4 alkyl" means a monovalent unsubstituted saturated straight-chain or branched-chain hydrocarbon radical having from 1 to 4 carbon atoms. Thus the term "C1-C4 alkyl" includes, for example, methyl, ethyl, n-propyl, isopropyl, n-butyl, isobutyl, sec-butyl and tert-butyl.

With respect to compounds of formula (IH), the term "C1-C4 alkoxy" means a monovalent unsubstituted saturated straight-chain or branched-chain hydrocarbon radical having from 1 to 4 carbon atoms linked to the point of substitution by a divalent O radical. Thus the term "C1-C4 alkoxy" includes, for example, methoxy, ethoxy, n-propoxy, isopropoxy, n-butoxy, isobutoxy, sec-butoxy and tert-butoxy.

With respect to compounds of formula (IH), the term "C1-C4 alkylthio" means a monovalent unsubstituted saturated straight-chain or branched-chain hydrocarbon radical having from 1 to 4 carbon atoms linked to the point of substitution by a divalent S radical. Thus the term "C1-C4 alkylthio" includes, for example, methylthio, ethylthio, n-propylthio, isopropylthio, n-butylthio, isobutylthio, sec-butylthio and tert-butylthio.

With respect to compounds of formula (IH), the term "C3-C6 cycloalkyl" means a monovalent unsubstituted saturated cyclic hydrocarbon radical having from 3 to 6 carbon atoms. Thus the term "C3-C6 cycloalkyl" includes, for example, cyclopropyl, cyclobutyl, cyclopentyl and cyclohexyl.

With respect to compounds of formula (IH), the term "C4-C7 cycloalkylalkyl" means a monovalent unsubstituted saturated cyclic hydrocarbon radical having from 3 to 6 carbon atoms linked to the point of substitution by a divalent unsubstituted saturated straight-chain or branched-chain hydrocarbon radical having at least 1 carbon atom. Thus the term"C4-C7 cycloalkyl" includes, for example, cyclopropylmethyl, cyclopropylethyl, cyclobutylmethyl, cyclopentylmethyl and cyclohexylmethyl.

With respect to compounds of formula (IH), the phrase "wherein one C-C bond within the cycloalkyl moiety is optionally substituted by an O-C, S-C or C=C bond" means that either (i) any two adjacent carbon atoms within a cycloalkyl ring may be linked by a double bond rather than a single bond (with the number of substituents on each carbon atom being reduced accordingly), or that (ii) one of any two adjacent C atoms within a cycloalkyl ring (and any substituents thereon) may be replaced by an oxygen or sulphur atom. Examples of groups encompassed by this phrase when used in conjunction with the term C3-C6 cycloalkyl include, for example:

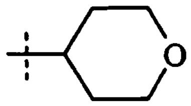 , 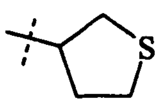 and 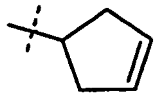 .

Examples of groups encompassed by this phrase when used in conjunction with the term C4-C7 cycloalkylalkyl include, for example:

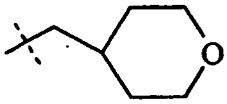 , 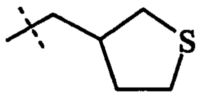 and 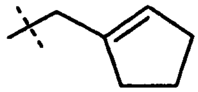 .

With respect to compounds of formula (IH), the term "C2-C6 alkenyl" means a monovalent unsubstituted unsaturated straight-chain or branched-chain hydrocarbon radical having from 2 to 6 carbon atoms and containing at least one carbon-carbon double bond. Thus the term "C1-C4 alkenyl" includes, for example, ethenyl, propenyl, 2-methyl-2-propenyl and butenyl.

With respect to compounds of formula (IH), the term "C3-C6 cycloalkoxy" means a monovalent unsubstituted saturated cyclic hydrocarbon radical having from 3 to 6 carbon atoms in the ring linked to the point of substitution by a divalent O radical. Thus the term "C3-C6 cycloalkoxyl" includes, for example, cyclopropoxy.

With respect to compounds of formula (IH), the term "C 1-C4 alkylsulfonyl" means a monovalent unsubstituted saturated straight-chain or branched-chain hydrocarbon radical having from 1 to 4 carbon atoms linked to the point of substitution by a divalent $SO_2$ radical. Thus the term "C1-C4 alkylsulfonyl" includes, for example, methylsulfonyl.

With respect to compounds of formula (IH), terms similar to the above definitions specifying different numbers of C atoms take an analogous meaning.

With respect to compounds of formula (IH), the term "halo" or "halogen" means F, Cl, Br or I.

With respect to compounds of formula (IH), the term "phenoxy" means a monovalent unsubstituted phenyl radical linked to the point of substitution by a divalent O radical.

With respect to compounds of formula (IH), the term "5-membered heteroaryl ring" means a 5-membered aromatic ring including one or more heteroatoms each independently selected from N, O and S. Preferably there are not more than three heteroatoms in total in the ring. More preferably there are not more than two heteroatoms in total in the ring. More preferably there is not more than one heteroatom in total in the ring. The term includes, for example, the groups thiazolyl, isothiazolyl, oxazolyl, isoxazolyl, thiophenyl, furanyl, pyrrolyl, imidazolyl, triazolyl, oxadiazolyl and thiadiazolyl.

"Thiazolyl" as used herein with respect to compounds of formula (IH) includes 2-thiazolyl, 4-thiazolyl and 5-thiazolyl.

"Isothiazolyl" as used herein with respect to compounds of formula (IH) includes 3-isothiazolyl, 4-isothiazolyl, and 5-isothiazolyl.

"Oxazolyl" as used herein with respect to compounds of formula (IH) includes 2-oxazolyl, 4-oxazolyl and 5-oxazolyl.

"Isoxazolyl" as used herein with respect to compounds of formula (IH) includes 3-isoxazolyl, 4-isoxazolyl, and 5-isoxazolyl.

"Thiophenyl" as used herein with respect to compounds of formula (IH) includes 2-thiophenyl and 3-thiophenyl.

"Furanyl" as used herein with respect to compounds of formula (IH) includes 2-furanyl and 3-furanyl.

"Pyrrolyl" as used herein with respect to compounds of formula (IH) includes 2-pyrrolyl and 3-pyrrolyl.

"Imidazolyl" as used herein with respect to compounds of formula (IH) includes 2-imidazolyl and 4-imidazolyl.

"Triazolyl" as used herein with respect to compounds of formula (IH) includes 1-triazolyl, 4-triazolyl and 5-triazolyl.

"Oxadiazolyl" as used herein with respect to compounds of formula (IH) includes 4-and 5-(1,2,3-oxadiazolyl);

3- and 5-(1,2,4-oxadiazolyl), 3-(1,2,5-oxadiazolyl), 2-(1,3,4-oxadiazolyl).

"Thiadiazolyl" as used herein with respect to compounds of formula (IH) includes 4-and 5-(1,2,3-thiadiazolyl), 3- and 5-(1,2,4-thiadiazolyl), 3-(1,2,5-thiadiazolyl), 2-(1,3,4-thiadiazolyl).

With respect to compounds of formula (IH), the term "6-membered heteroaryl ring" means a 6-membered aromatic ring including one or more heteroatoms each independently selected from N, O and S. Preferably there are not more than three heteroatoms in total in the ring. More preferably there are not more than two heteroatoms in total in the ring. More preferably there is not more than one heteroatom in total in the ring. The term includes, for example, the groups pyridyl, pyrimidyl, pyrazinyl, pyridazinyl and triazinyl.

"Pyridyl" as used herein with respect to compounds of formula (IH) includes 2-pyridyl, 3-pyridyl and 4-pyridyl.

"Pyrimidyl" as used herein with respect to compounds of formula (IH) includes 2-pyrimidyl, 4-pyrimidyl and 5-pyrimidyl.

"Pyrazinyl" as used herein with respect to compounds of formula (IH) includes 2-pyrazinyl and 3-pyrazinyl.

"Pyridazinyl" as used herein with respect to compounds of formula (IH) includes 3-pyridazinyl and 4-pyridazinyl.

"Triazinyl" as used herein with respect to compounds of formula (IH) includes 2-(1,3,5-triazinyl), 3-, 5- and 6-(1,2,4-triazinyl) and 4- and 5-(1,2,3-triazinyl).

With respect to compounds of formula (IH), the term "*ortho*" refers to a position on the Ar1 aromatic ring which is adjacent to the position from which Ar1 links to the rest of the compound of formula (IH).

Preferred compounds of formula (IH) are those wherein X is OH, C1-C4 alkoxy, or $NH_2$. More preferably, X is OH or $NH_2$. Most preferably X is OH.

Preferred compounds of formula (IH) are those wherein Rx is H or methyl. Most preferably Rx is H.

Preferred compounds of formula (IH) are those wherein Ry is H or methyl. Most preferably Ry is H.

Preferred compounds of formula (IH) are those wherein each Rz group is independently H or methyl, with the proviso that not more than 3 Rz groups may be methyl. Most preferably, each Rz is H.

Preferred compounds of formula (IH) are those wherein R1 is C1-C6 alkyl (optionally substituted with 1, 2 or 3 halogen atoms and/or with 1 substituent selected from C1-C4 alkylthio (optionally substituted with 1, 2 or 3 fluorine atoms), C1-C4 alkoxy (optionally substituted with 1, 2 or 3 fluorine atoms), C3-C6 cycloalkoxy, C1-C4 alkylsulfonyl, cyano, -CO-O(C1-C2 alkyl), -O-CO-(C1-C2 alkyl) and hydroxy). More preferably, R1 is C1-C6 alkyl (optionally substituted with 1,2 or 3 halogen atoms and/or with 1 substituent selected from C1-C4 alkoxy (optionally substituted with 1, 2 or 3 fluorine atoms), cyano and hydroxy). More preferably, R1 is C1-C6 alkyl (optionally substituted with 1, 2 or 3 halogen atoms). More preferably, R1 is C1-C6 alkyl (optionally substituted with 1, 2 or 3 fluorine atoms). Examples of specific identities for R1 within this embodiment include methyl, ethyl, iso-propyl, iso-butyl, 3,3,3-trifluoropropyl and 4,4,4-trifluorobutyl.

Preferred compounds of formula (IH) are those wherein R1 is C2-C6 alkenyl (optionally substituted with 1, 2 or 3 halogen atoms).

Preferred compounds of formula (IH) are those wherein R1 is C3-C6 cycloalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C, S-C or C=C bond. More preferably, R1 is C3-C6 cycloalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C bond. More preferably, R1 is C3-C6 cycloalkyl wherein one C-C bond within the cycloalkyl moiety is optionally substituted by an O-C bond. Examples of specific identities for R1 within this embodiment include cyclopropyl, cyclopentyl and tetrahydropyranyl (in particular tetrahydro-2H-pyran-4-yl).

Preferred compounds of formula (IH) are those wherein R1 is C4-C7 cycloalkylalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C, S-C or C=C bond.

Preferred compounds of formula (IH) are those wherein R1 is $CH_2$Ar2 wherein Ar2 is as defined above. More preferably, R1 is $CH_2$Ar2 wherein Ar2 is a phenyl ring or a pyridyl (preferably 2-pyridyl) ring each of which may be substituted with 1, 2 or 3 substituents each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo and hydroxy. More preferably, R1 is $CH_2$Ar2 wherein Ar2 is a phenyl ring optionally substituted in the manner described in the preceding sentence. More preferably, R1 is $CH_2$Ar2 wherein Ar2 is a phenyl ring optionally substituted with 1 or 2 substituents each independently selected from C1-C4 alkyl (optionally substituted with 1, 2 or 3 halogen atoms), C1-C4 alkoxy (optionally substituted with 1, 2 or 3 halogen atoms), halo and hydroxy. Examples of specific identities for R1 within this embodiment include phenylmethyl and (2-methoxy-phenyl) methyl.

Preferred compounds of formula (IH) are those wherein Ar1 is a phenyl ring or a 5- or 6-membered heteroaryl ring; each of which is substituted in the *ortho* position with a substituent selected from C1-C4 alkyl (optionally substituted with 1, 2 or 3 halogen atoms), C1-C4 alkoxy (optionally substituted with 1, 2 or 3 halogen atoms), C1-C4 alkylthio

(optionally substituted with 1, 2 or 3 halogen atoms), -CO-O(C1-C4 alkyl), cyano, -NRR, -CONRR, halo, hydroxy, pyridyl, thiophenyl, phenyl, benzyl and phenoxy, each of which *ortho* substituents is optionally ring-substituted (where a ring is present) with 1, 2 or 3 substituents each independently selected from halogen, C1-C4 alkyl (optionally substituted with 1, 2 or 3 halogen atoms), C1-C4 alkoxy (optionally substituted with 1, 2 or 3 halogen atoms), carboxy, nitro, hydroxy, cyano, -NRR, - CONRR, $SO_2NRR$ and $SO_2R$; and each of which is (in addition to *ortho* substitution) optionally further substituted with 1 or 2 substituents each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), -CO-O(C1-C4 alkyl), cyano, -NRR, -CONRR, halo and hydroxy. More preferably, Ar1 is a phenyl ring or a pyridyl (preferably 2-pyridyl) ring each of which is substituted and optionally further substituted in the manner described in the preceding sentence. More preferably, Ar1 is a group of the formula (a):

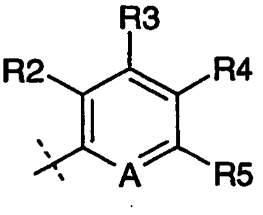
(a)

wherein,

A is N or CR6 (preferably CR6); R2 is C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo, hydroxy, pyridyl, thiophenyl, phenyl (optionally substituted with 1, 2 or 3 substituents each independently selected from halogen, C1-C4 alkyl (optionally substituted with 1, 2 or 3 halogen atoms), or C1-C4 alkoxy (optionally substituted with 1, 2 or 3 halogen atoms)) or phenoxy (optionally substituted with 1, 2 or 3 halogen atoms); R3 is H; R4 is H; R5 is H, C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo or hydroxy; and R6 (if present) is H. More preferably, Ar1 is a group of the formula (a) wherein, A is CR6; R2 is C1-C4 alkyl (optionally substituted with 1, 2 or 3 fluorine atoms), C1-C4 alkoxy (optionally substituted with 1, 2 or 3 fluorine atoms) or phenyl (optionally substituted with 1, 2 or 3 fluorine atoms); R3 is H; R4 is H; R5 is H or F; and R6 is H. Examples of specific identities for Ar1 include 2-methoxy-phenyl, 2-ethoxy-phenyl, 2-trifluoromethoxy-phenyl, 2-phenyl-phenyl, 2-(3-fluoro-phenyl)-phenyl, 2-methoxy-5-fluoro-phenyl and 2-phenyl-5-fluoro-phenyl.

It will be appreciated that a compound of formula (IH) above will possess at least two asymmetric carbon atoms. For compounds of formula (IH), where a structural formula does not specify the stereochemistry at one or more chiral centres, it encompasses all possible stereoisomers and all possible mixtures of stereoisomers (including, but not limited to, racemic mixtures), which may result from stereoisomerism at each of the one or more chiral centers. Preferred compounds of formula (IH) are those of formula (IIH)

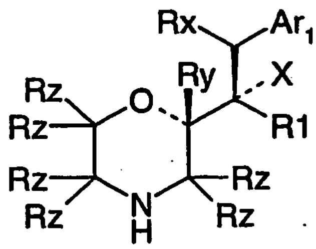

(IIIH)

wherein, X, Rx, Ry, Rz, R1 and Ar1 are as defined for formula (I) above; or a pharmaceutically acceptable salt thereof.

Preferred compounds of formula (IH) are those of formula (IIIH)

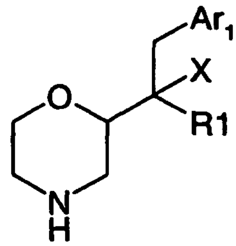

(IIIH)

wherein, X, R1 and Ar1 are as defined for formula (IH) above; or a pharmaceutically acceptable salt thereof.

Preferred compounds of formula (IH) are those of formula (IIIH) wherein X is OH or $NH_2$;

R1 is C1-C6 alkyl (optionally substituted with 1, 2 or 3 halogen atoms and/or with 1 substituent selected from C1-C4 alkylthio (optionally substituted with 1, 2 or 3 fluorine atoms), C1-C4 alkoxy (optionally substituted with 1, 2 or 3 fluorine atoms), C3-C6 cycloalkoxy, C1-C4 alkylsulfonyl; cyano, -CO-O(C1-C2 alkyl), -O-CO-(C1-C2 alkyl) and hydroxy); C3-C6 cycloalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C, S-C or C=C bond; or $CH_2$Ar2 wherein Ar2 is a phenyl ring or a pyridyl (preferably 2-pyridyl) ring each of which may be substituted with 1, 2 or 3 substituents each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo and hydroxy; and

Ar1 is a phenyl ring or a 5- or 6-membered heteroaryl ring; each of which is substituted in the *ortho* position with a substituent selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), -CO-O(C1-C4 alkyl), cyano, -NRR, -CONRR, halo, hydroxy, pyridyl, thiophenyl, phenyl, benzyl and phenoxy, each of which *ortho* substituents is optionally ring-substituted (where a ring is present) with 1, 2 or 3 substituents each independently selected from halogen, C1-C4 alkyl (optionally substituted with 1, 2 or 3 halogen atoms), C1-C4 alkoxy (optionally substituted with 1, 2 or 3 halogen atoms), carboxy, nitro, hydroxy, cyano, - NRR, -CONRR, $SO_2$NRR and $SO_2$R; and each of which is (in addition to *ortho* substitution) optionally further substituted with 1 or 2 substituents each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), -CO-O(C1-C4 alkyl), cyano, -NRR, -CONRR, halo and hydroxy; or a pharmaceutically acceptable salt thereof.

Preferred compounds of formula (IH) are those of formula (IVH)

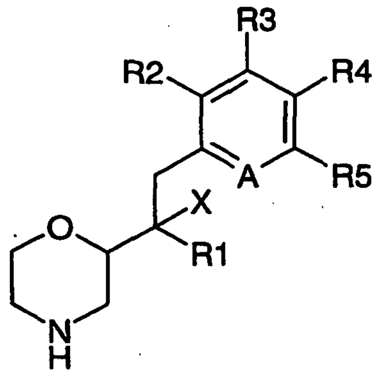

(IVH)

wherein,

X is OH or $NH_2$;

R1 is C1-C6 alkyl (optionally substituted with 1, 2 or 3 halogen atoms and/or with 1 substituent selected from C1-C4 alkoxy (optionally substituted with 1, 2 or 3 fluorine atoms), cyano, and hydroxy); C3-C6 cycloalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C bond; or $CH_2Ar2$ wherein Ar2 is a phenyl ring optionally substituted with 1, 2 or 3 substituents each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo and hydroxy;
A is N or CR6 (preferably CR6); R2 is C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo, hydroxy, pyridyl, thiophenyl, phenyl (optionally substituted with 1, 2 or 3 substituents each independently selected from halogen, C1-C4 alkyl (optionally substituted with 1, 2 or 3 halogen atoms), or C1-C4 alkoxy (optionally substituted with 1, 2 or 3 halogen atoms)) or phenoxy (optionally substituted with 1, 2 or 3 halogen atoms); R3 is H; R4 is H; R5 is H, C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo or hydroxy; and R6 (if present) is H; or a pharmaceutically acceptable salt thereof.

Preferred compounds of formula (IH) are those of formula (VH)

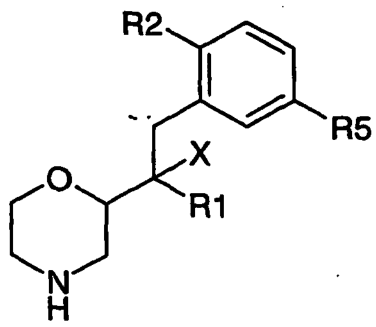

(VH)

wherein,

X is OH or $NH_2$;
R1 is C1-C6 alkyl (optionally substituted with 1,2 or 3 fluorine atoms), C3-C6 cycloalkyl wherein one C-C bond within the cycloalkyl moiety is optionally substituted by an O-C bond or $CH_2Ar2$ wherein At2 is a phenyl ring optionally substituted with 1 or 2 substituents each independently selected from C1-C4 alkyl (optionally substituted with 1, 2 or 3 halogen atoms), C1-C4 alkoxy (optionally substituted with 1, 2 or 3 halogen atoms), halo and hydroxy;
R2 is C1-C4 alkyl (optionally substituted with 1, 2 or 3 fluorine atoms), C1-C4 alkoxy (optionally substituted with 1, 2 or 3 fluorine atoms) or phenyl (optionally substituted with 1, 2 or 3 fluorine atoms); and R5 is H or F; or a pharmaceutically acceptable salt thereof.

Preferred compounds of formula (IH) are those of formula (VIH)

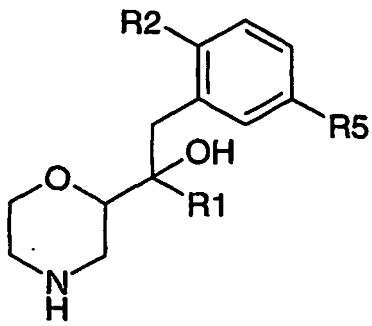

(VIH)

wherein,

R1 is C1-C6 alkyl (optionally substituted with 1, 2 or 3 fluorine atoms) or C3-C6 cycloalkyl wherein one C-C bond within the cycloalkyl moiety is optionally substituted by an O-C bond;
R2 is C1-C4 alkyl (optionally substituted with 1, 2 or 3 fluorine atoms), C1-C4 alkoxy (optionally substituted with 1, 2 or 3 fluorine atoms) or phenyl (optionally substituted with 1, 2 or 3 fluorine atoms); and R5 is H or F; or a pharmaceutically acceptable salt thereof.

Compounds within the scope of Formula (IH) above are selective inhibitors of norepinephrine reuptake. Biogenic amine transporters control the amount of biogenic amine neurotransmitters in the synaptic cleft. Inhibition of the respective transporter leads to a rise in the concentration of that neurotransmitter within the synaptic cleft. Compounds of Formula (IH) above and their pharmaceutically acceptable salts preferably exhibit a $K_i$ value less than 1000nM, more preferably less than 500nM, at the norepinephrine transporter as determined using the scintillation proximity assay as described below. More preferred compounds of Formula (IH) above and their pharmaceutically acceptable salts exhibit a $K_i$ value less than 100nM at the norepinephrine transporter. More preferred compounds of Formula (IH) above and their pharmaceutically acceptable salts exhibit a $K_i$ value less than 50nM at the norepinephrine transporter. Especially preferred compounds of Formula (IH) above and their pharmaceutically acceptable salts exhibit a $K_i$ value less than 20nM at the norepinephrine transporter. Preferably, these compounds selectively inhibit the norepinephrine transporter relative to the serotonin and dopamine transporters by a factor of at least five, more preferably by a factor of at least ten.

In addition, the compounds of Formula (IH) above of the present invention are preferably acid stable. Advantageously, they have a reduced interaction (both as substrate and inhibitor) with the liver enzyme Cytochrome P450 (CYP2D6). That is to say, they preferably exhibit less than 75% metabolism via the CYP2D6 pathway according to the CYP2D6 substrate assay described below and they preferably exhibit an IC50 of >6μM according to the CYP2D6 inhibitor assay described below.

Norepinephrine reuptake inhibitors useful in the present invention are selective for the reuptake of norepinephrine over the reuptake of other neurotransmitters, e.g. serotonin and dopamine. It is also preferred that the norepinephrine reuptake inhibitor does not exhibit significant direct agonist or antagonist activity at other receptors.

The present invention encompasses the use of pharmaceutical compositions comprising the compounds disclosed herein, or pharmaceutically acceptable salts thereof, together with a pharmaceutically acceptable carrier, diluent, or excipient.

It will be understood by the skilled reader that most or all of the compounds used in the present invention are capable of forming salts, and that the salt forms of pharmaceuticals are commonly used, often because they are more readily crystallized and purified than are the free bases. In all cases, the use of the pharmaceuticals described above as salts is contemplated in the description herein, and often is preferred, and the pharmaceutically acceptable salts of all of the compounds are included in the names of them.

Many of the compounds used in this invention are amines, and accordingly react with any of a number of inorganic and organic acids to form pharmaceutically acceptable acid addition salts. Since some of the free amines of the compounds of this invention are typically oils at room temperature, it is preferable to convert the free amines to their pharmaceutically acceptable acid addition salts for ease of handling and administration, since the latter are routinely solid at room temperature. Acids commonly employed to form such salts are inorganic acids such as hydrochloric acid, hydrobromic acid, hydroiodic acid, sulfuric acid, phosphoric acid, and the like, and organic acids, such as p-toluenesulfonic acid, methanesulfonic acid, oxalic acid, p-bromophenylsulfonic acid, carbonic acid, succinic acid, citric acid, benzoic acid, acetic acid and the like. Examples of such pharmaceutically acceptable salts thus are the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, b-hydroxybutyrate, glycollate, tartrate, methanesulfonate, propanesulfonate, naphthalene-1-sulfonate, naphthalene-2-sulfonate, mandelate and the like. Preferred pharmaceutically acceptable salts are those formed with hydrochloric acid.

Pharmaceutically acceptable salts of the compounds of Formula (IH) above include acid addition salts, including salts formed with inorganic acids, for example hydrochloric, hydrobromic, nitric, sulphuric or phosphoric acids, or with organic acids, such as organic carboxylic or organic sulphonic acids, for example, acetoxybenzoic, citric, glycolic, o-mandelic-1, mandelic-dl, mandelic d, maleic, mesotartaric monohydrate, hydroxymaleic, fumaric, lactobionic, malic,

methanesulphonic, napsylic, naphtalenedisulfonic, naphtoic, oxalic, palmitic, phenylacetic, propionic, pyridyl hydroxy pyruvic, salicylic, stearic, succinic, sulphanilic, tartaric, 2-hydroxyethane sulphonic, toluene-p-sulphonic, and xinafoic acids.

In addition to the pharmaceutically acceptable salts, other salts can serve as intermediates in the purification of compounds, or in the preparation of other, for example pharmaceutically acceptable, acid addition salts, or are useful for identification, characterization, or purification.

The present invention encompasses the administration of a composition that exhibits selective norepinephrine reuptake inhibitor activity. The composition can comprise one or more agents that, individually or together, inhibit norepinephrine reuptake in a selective manner.

The dosages of the drugs used in the present invention must, in the final analysis, be set by the physician in charge of the case using knowledge of the drugs, the properties of the drugs in combination as determined in clinical trials, and the characteristics of the patient including diseases other than that for which the physician is treating the patient. General outlines of the dosages, and some preferred dosages, are from 5 to 500 mg, more preferably from 25 to 300 mg, of the active ingredient per patient per day.

The pharmaceutical compositions are prepared in a manner well known in the pharmaceutical art. The carrier or excipient can be a solid, semi-solid, or liquid material that can serve as a vehicle or medium for the active ingredient. Suitable carriers or excipients are well known in the art. The pharmaceutical composition can be adapted for oral, inhalation, parenteral, or topical use, and can be administered to the patient in the form of tablets, capsules, aerosols, inhalants, suppositories, solutions, suspensions, or the like.

The compounds useful for the medicaments of the present invention can be administered orally, for example, with an inert diluent or capsules or compressed into tablets. For the purpose of oral therapeutic administration, the compounds can be incorporated with excipients and used in the form of tablets, troches, capsules, elixirs, suspensions, syrups, wafers, chewing gums, and the like. These preparations should contain at least 4% of the compound of the present invention, the active ingredient, but can be varied depending upon the particular form and may conveniently be between 4% to about 70% of the weight of the unit. The amount of the compound present in compositions is such that a suitable dosage will be obtained. Preferred compositions and preparations useful for the medicaments of the present invention can be determined by a person skilled in the art.

The tablets, pills, capsules, troches, and the like can also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it can contain, in addition to materials of the above type, a liquid carrier such as polyethylene glycol or a fatty oil. Other dosage unit forms can contain other various materials that modify the physical form of the dosage unit, for example, as coatings. Thus, tablets or pills can be coated with sugar, shellac, or other coating agents. A syrup can contain, in addition to the present compounds, sucrose as a sweetening agent and certain preservatives, dyes and colorings, and flavors. Materials used in preparing these various compositions should be pharmaceutically pure and non-toxic in the amounts used.

For the purpose of parenteral therapeutic administration, the compounds of use in the present invention can be incorporated into a solution or suspension. These preparations typically contain at least 0.1% of a compound of the invention, but can be varied to be between 0.1 and 90% of the weight thereof. The amount of the compound present in such compositions is such that a suitable dosage will be obtained. The solutions or suspensions can also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetra-acetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Preferred compositions and preparations can be determined by one skilled in the art.

## Inhibition of Norepinephrine Reuptake

The ability of compounds to inhibit the reuptake of norepinephrine can be measured by the general procedure of Wong, *et al., supra*.

Male Sprague-Dawley rats weighing 150-250 gm are decapitated and brains are immediately removed. Cerebral cortices are homogenized in 9 volumes of a medium containing 0.32 M sucrose and 10 mM glucose. Crude synaptosomal preparations are isolated after differential centrifugation at 1000 x g for 10 minutes and 17,000 x g for 28 minutes. The final pellets are suspended in the same medium and kept in ice until use within the same day.

Synaptosomal uptake of $^3$H-norepinephrine is determined as follows. Cortical synaptosomes (equvalent to 1 mg of protein) are incubated at 37°C for 5 minutes in 1 mL Krebs-bicarbonate medium containing also 10 mM glucose,

0.1 mM iproniazide, 1 mM ascorbic acid, 0.17 mM EDTA and 50 nM $^3H$-norepinephrine. The reaction mixture is immediately diluted with 2 mL of ice-chilled Krebs-bicarbonate buffer and filtered under vacuum with a cell harvester (Brandel, Gaithersburg, MD). Filters are rinsed twice with approximately 5 mL of ice-chilled 0.9% saline and the uptake of $^3H$-norepinephrine assessed by liquid scintillation counting. Accumulation of $^3H$-norepinephrine at 4°C is considered to be background and is subtracted from all measurements. The concentration of the test compound required to inhibit 50% of the $^3H$-norepinephrine accumulation ($IC_{50}$ values) are determined by linear regression analysis.

It should be noted that the medicaments of the present invention are effective in the treatment of children, adolescents, and adults. For purposes of the present invention, a child is considered to be a patient below the age of puberty, an adolescent is considered to be a patient from the age of puberty up to 18 years of age, and an adult is considered to be a patient 18 years or older.

**Preparation of Compounds of Formula (IH)**

Compounds of formula (IH) may be prepared by conventional organic chemistry techniques. General schemes outlining the synthetic routes to compounds of formula (IH) are described below. For clarity, Rx, Ry and Rz are shown as H, however, it will be appreciated that analogous methods could be applied for other possible identities of Rx, Ry and Rz.

The key intermediates of formulae (XH), (XIH) and (XIIH) may be prepared as shown below (where P represents an N-protecting group):

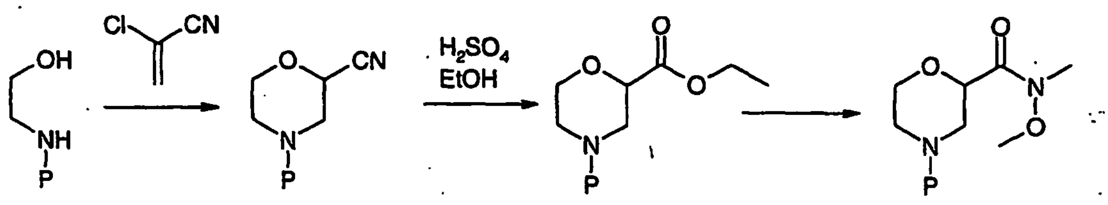

(XH) (XIH) (XIIH)

N-protected ethanolamine is reacted with 2-chloroacrylonitrile to give a Michael adduct which is then treated *in situ* with a base, such as potassium t-butoxide, to give a compound of formula (XH). The compound of formula (XH) may then be hydrolysed in $H_2SO_4$/ethanol to give the ester of formula (XIH). This in turn may be converted into the Weinreb amide of formula (XIIH) by adding a solution of (XIH) to a premixed solution of *N,N*-dimethylhydroxylamine and trimethylaluminium. Suitable N-protecting groups will be known to the person skilled in the art. Further information on suitable N-protecting groups is contained in the well known text "Protective Groups in Organic Synthesis", Theodora W. Greene and Peter G.M. Wuts, John Wiley & Sons, Inc., New York, 1999, pp.494-653. Benzyl is an especially preferred N-protecting group.

N-protected compounds of formula (IH) wherein X is $NH_2$ may be prepared from compounds of formula (XH) as shown below:

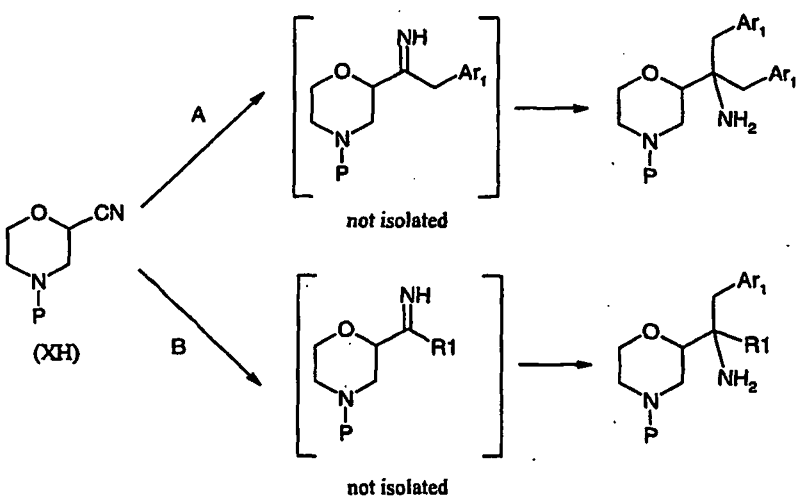

(XH)

In route A the intermediate (XH) is treated with an excess of the Grignard reagent $Ar1CH_2MgBr$ to provide an N-protected compound of formula (IH) wherein X is $NH_2$ and R1 is $CH_2Ar2$ wherein Ar2 = Ar1. In route B the intermediate (XH) is treated with one equivalent of the Grignard reagent R1MgBr followed by one equivalent of the Grignard reagent $Ar1CH_2MgBr$ to provide an N-protected compound of formula (IH) wherein X is $NH_2$. Alternatively, the Grignard reagent $ArICH_2MgBr$ may be added first followed by R1MgBr. Preferably, a Lewis acid such as titanium isopropoxide is added to the reaction mixture in between addition of the Grignard reagents (see Charette, A.B.; Gagnon, A; Janes, M; Mellon, C; Tetrahedron Lett, 1998, 39(29), 5147-5150 and Charette, A.B.; Gagnon, A; Tetrahedron: Asymmetry, 1999, 10(10), 1961-1968).

N-protected compounds of formula (IH) wherein X is OH and R1 is CHAr2 wherein Ar2 = Ar1 may be prepared from compounds of formula (XIH) as shown below:

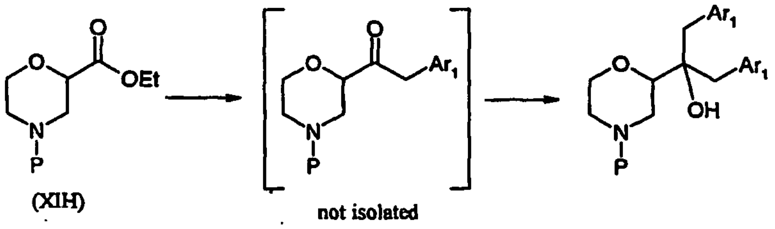

(XIH)

not isolated

Intermediate (XIH) is treated with an excess of the Grignard reagent $Ar1CH_2MgBr$ to provide an N-protected compound of formula (IH) wherein X is OH and R1 is $CH_2Ar2$ wherein Ar2 = Ar1.

N-protected compounds of formula (IH) wherein X is OH may be prepared from the Weinreb amide of formula (XIIH) as shown below: .

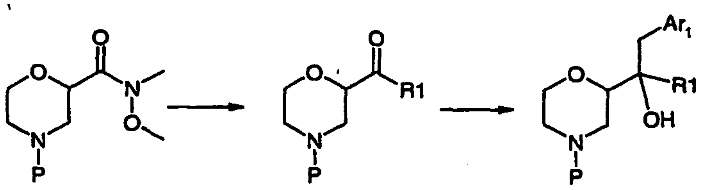

(XIIH) (XIIIH)

To a solution of (XIIH) is added a solution of the requisite Grignard reagent R1MgBr to provide, on work up, a compound of formula (XIIIH). To a solution of the ketone of formula (XIIIH) is added a solution of the Grignard reagent $Ar1CH_2MgBr$ to provide an N-protected compound of formula (IH) wherein X is OH.

The ketones of formula (XIIIH) may also be obtained via a different route as shown below:

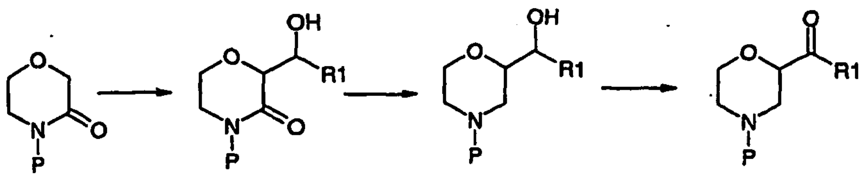

(XIIIH)

A solution of N-protected morpholinone is treated with a strong base such as lithium diisopropylamide. To this solution is added an aldehyde R1CHO. Reduction of the morpholine carbonyl group using, for example, borane-THF complex followed by oxidation of the alcohol using, for example, Swern oxidation conditions, provides a compound of formula (XIIIH) which can be reacted onward as described in the previous scheme to provide an N-protected compound of formula (IH) wherein X is OH.

N-protected compounds of formula (IH) wherein X is C1-C4 alkoxy, may be synthesized by standard alkylation of the N-protected compounds of formula (IH) wherein X=OH as shown below:

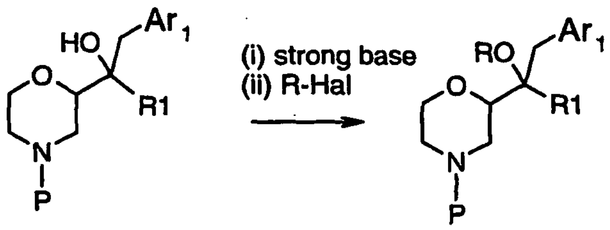


Suitable strong bases will be known to the person skilled in the art and include, for example, sodium hydride. Similarly, suitable alkylating agents will be known to the person skilled in the art and include, for example, C1-C4 alkyl halides such as methyl iodide.

N-protected compounds of formula (IH) wherein X is NH(C1-C4 alkyl), may be synthesized by treatment of a compound of formula (IH) wherein X = $NH_2$ under reductive alkylating conditions or using suitable alkylating agents known to the person skilled in the art including, for example, C1-C4 alkyl halides such as methyl iodide.

N-protected compounds of the present invention may be elaborated upon using standard organic chemistry to provide further N-protected compounds of formula (IH). For example, organometallic type couplings between an Ar1-Br derivative and a phenylboronic acid as shown below can provide Ar1-phenyl derivatives.

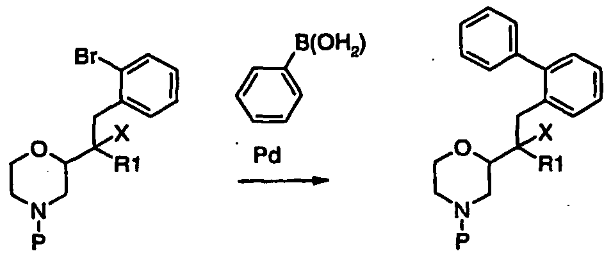


Compounds of formula (IH) may be obtained by deprotection of the N-protected intermediates as shown below:

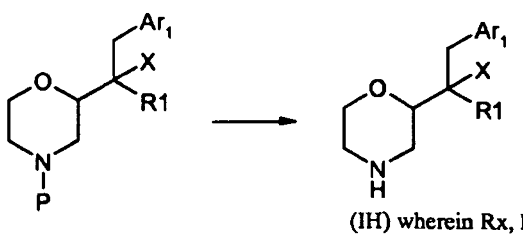

(IH) wherein Rx, Ry and Rz = H

Further information on suitable deprotection methods is contained in the well known text "Protective Groups in Organic Synthesis" referenced above.

It will be appreciated that compounds of Formula (IH) above possess one or more asymmetric carbon atoms, and that in the present invention specific individual stereoisomers are preferred. In the present specification, where a structural formula does not specify the stereochemistry at one or more chiral centres, it encompasses all possible stereoisomers and all possible mixtures of stereoisomers (including, but not limited to, racemic mixtures), which can result from stereoisomerism at each of the one or more chiral centers.

The following examples illustrate compounds of of Formulae (IH) above and methods for their preparation.

Examples of compounds of formula (IH) may be prepared by conventional organic chemistry techniques from *N*-benzyl-morpholine-2-carboxylic acid ethyl ester **1** as outlined in **Scheme 1H.**

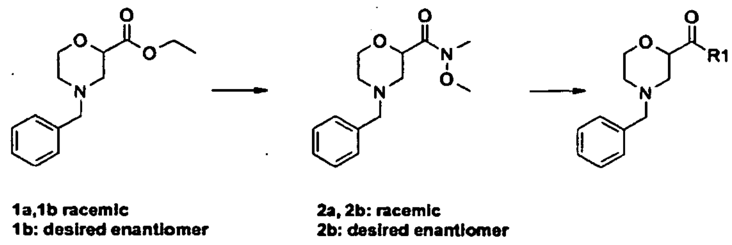


**Scheme 1H**

Conversion of **1** into Weinreb amide **2** followed by treatment with a suitable Grignard reagent leads to ketones listed in **Table 1H.**

**Table 1H**

| R1 | Number |
|---|---|
| methyl | 3 |
| ethyl. | 4 |
| isopropyl | 5 |
| isobutyl | 6 |
| cyclopentyl | 7 |
| tetrahydropyranyl | 8 |
| 3,3,3-trifluoropropyl | 9 |
| 4,4,4-trifluorobutyl | 10 |
| cyclopropyl | 77 |

Cyclopropyl-substituted ketone 77 may alternatively be obtained from N-benzyl morpholinone as outlined in **Scheme 4H**.

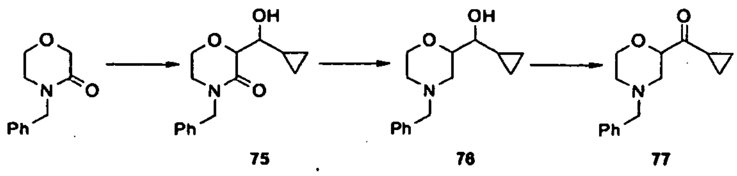


**Scheme 4H**

Treatment of the benzyl-morpholinine with a strong base such as lithium diisopropylamide followed by addition of cyclopropyl methylaldehyde gives **75.** Reduction of **75** with, for example, borane-THF complex gives **76.** Addition of a solution of **76** to a pre-mixed solution of dimethylsulfoxide and oxalyl chloride provides **77.**

Reaction of ketones listed in **Table 1H** with a suitably substituted benzyl Grignard reagent gives N-benzyl substituted tertiary alcohols listed in **Table 2H** as outlined in **Scheme 2H**.

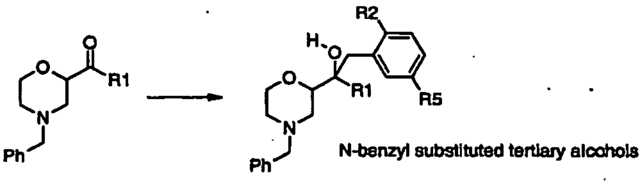


**Scheme 2H**

Debenzylation and salt formation as detailed in **Scheme 3H** leads to the tertiary alcohol salts listed in **Table 2H.**

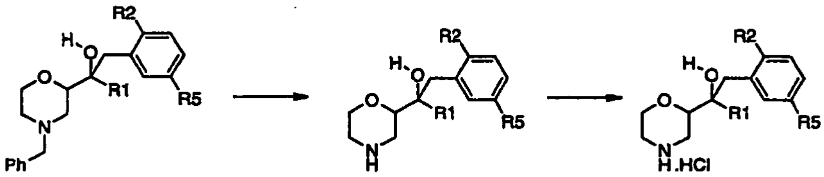

**Scheme 3H**

**Table 2H**

| Example | R1 | R2,R5 | N-Benzyl | HCl Salt |
|---|---|---|---|---|
| 1H | methyl | 2-Ph | 11 | 12 |
| 2H | ethyl | 2-OMe,5-F | 13 | 14 |
| 3H | ethyl | 2-OCF3 | 15 | 16 |
| 4H | ethyl | 2-Ph | 17 | 18 |
| 5H | isopropyl | 2-OMe, 5-F | 19 | 20 |
| 6H | isopropyl | 2-OMe | 21 | 22 |
| 7H | isopropyl | 2-Oet | 23 | 24 |
| 8H | isopropyl | 2-OCF3 | 25 | 26 |
| 9H | isopropyl | 2-Ph | 27 | 28 |
| 10H | isopropyl | 2-Ph,5-F | 29 | 30 |
| 11H | isobutyl | 2-OMe, 5-F | 31 | 32 |
| 12H | isobutyl | 2-Oet | 33 | 34 |
| 13H | isobutyl | 2-OCF3 | 35 | 36 |
| 14H | isobutyl | 2-Ph | 37 | 38 |
| 15H | isobutyl | 2-Ph, 5-F | 39 | 40 |
| 16H | cyclopentyl | 2-OMe, 5-F | 41 | 42 |
| 17H | cyclopentyl | 2-Oet | 43 | 44 |
| 18H | cyclopentyl | 2-OCF3 | 45 | 46 |
| 19H | cyclopentyl | 2-Ph | 47 | 48 |
| 20H | cyclopentyl | 2-Ph, 5-F | 49 | 50 |
| 21H | tetrahydropyranyl | 2-OMe, 5-F | 51 | 52 |
| 22H | tetrahydropyranyl | 2-OCF3 | 53 | 54 |

Examples **1H** to **22H** can be otained in enantiomerically pure form via this route using chirally pure ester **1b.** Resolution of **1** into **1a** and **1b** can be achieved through chiral HPLC. Addition of the benzyl Grignard reagent is a stereoselective process and gives predominantly one diastereomer with only small amounts of the second diastereomer. No epimerisation was observed during removal of the benzyl group. Alternatively, enantiomerically pure products are obtained through conversion to an N-protected analogue such as butyloxycarbonyl or carbobenzyloxy followed by separation by chiral HPLC. Removal of the N-protecting group leads to enantiomerically highly enriched products.

**General Synthetic Procedures for the Preparation of Examples 1H to 22H**

*General Procedure* 1: Preparation of N-benzvl morpholine alkyl ketones

To a solution of the carboxamide **2** or **2b** in anhydrous THF at 0°C is added a solution of the requisite Grignard reagent (1.2-3 eq in one or two aliquots). The reaction mixture is allowed to warm up to room temperature and left stirring for 45 minutes to 2 hours before quenching either with 1M hydrochloric acid or saturated ammonium chloride solution and extracting either in DCM or ethyl acetate. The combined organic layers are dried over magnesium sulphate, filtered and concentrated *in vacuo* to give the corresponding alkyl ketones **3-10** and **77**.

*General Procedure 2:* Preparation of N-benzyl tertiary alcohols

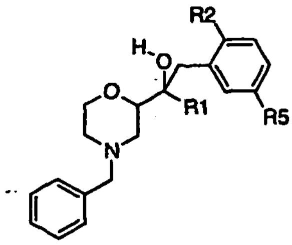

To a solution of the ketones **3-10** and **77** in anhydrous THF at 0°C is added a solution of the requisite benzyl Grignard reagent (1.1-1.5 eq). The reaction mixture is allowed to warm up to room temperature and left stirring for 1-2 hours before quenching by addition of cold water. After extraction of the aqueous layer in DCM, the combined organic layers are washed with brine, dried over magnesium sulphate, filtered and concentrated *in vacuo* to give the title N-benzyl tertiary alcohols. Purification details are listed for individual compounds.

*General Procedure 3:* Debenzylation ofN-benzyl tertiary alcohols

To a solution of the requisite N-benzyl tertiary alcohol in anhydrous DCM is added solid supported Hünig's base (Argonaut, 3.56 mmol/g, 2-4 eq) and α-chloroethyl chloroformate (3 to 10 eq) at room temperature under nitrogen. The reaction mixture is heated to 40°C and the reaction is monitored by $FIA^+$ and LCMS analysis. After completion the reaction mixture is filtered, and the resin washed with DCM. The combined organic phases are concentrated *in vacuo.* Methanol is added and the solution heated to 60°C for 1.5 to 8 hours. After complete consumption of starting material the methanol solution is evaporated to give a product, which is further purified as detailed for individual compounds.

*General Procedure 4:* Conversion of amines into hydrochloride salts

To a solution of the requisite amine in dry diethyl ether (5-10 ml) is added hydrochloric acid (1.2 eq, 1M solution in diethyl ether). Ether is blown off with a stream of nitrogen or removed *in vacuo* and the samples were either dried under high vacuum for several hours or freeze-dried (acetonitrile/water 1:1 [v/v]) to give the hydrochloride salts in near quantitative yield.

*General Procedure 5:* Preparation of benzyl Grignard reagents

Such reagents were prepared from the requisite benzyl halide using methods known to those skilled in the art (see for example Fieser, L.F. and Fieser, M.F. "Reagents for Organic Synthesis", John Wiley and Sons Inc., Vol. 1, pp. 415-424 or March, J. "Advanced Organic Chemistry", John Wiley and Sons Inc., 3rd Ed., pp. 558-561). The requisite

benzyl halides were either commercially available or prepared using previously published literature methods.

**Preparation of Intermediates for the Synthesis of Examples 1H-22H**

***4-Benzyl-morpholine-2-carbonitrile***

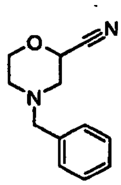

A one-litre reactor with mechanical stirring, cooled by an ice bath, is charged with *N*-benzylethanolamine (172.2 g; 1 equiv. available from Aldrich Chemical Company). 2-Chloroacrylonitrile (100 g; 1 equiv. available from Aldrich Chemical Company) is added dropwise over 2 minutes. The temperature is maintained between 23 °C and 29 °C by means of the ice bath and subsequently a water bath at 15 °C. After one night stirring at room temperature (water bath), the mixture is dissolved in tetrahydrofuran and transferred to a 2 L reactor which is cooled to -5°C by ice/NaCl bath. The total volume of tetrahydrofuran is 1.35 L. Potassium *tert*-butoxide (148 g; 1.1 equiv.) is added by portions over 1 hour, keeping the reaction temperature at 0±2 °C. After 1 hour post-stirring at 0 °C, the mixture is quenched with saturated $NaHCO_3$ (500 mL). The aqueous layer is extracted with diethyl ether (500 mL). Organic layers are dried over $MgSO_4$ and evaporated to dryness. The title compound (149.8 g; 65%) is obtained after percolation of the 250 g dry residue on 1 kg of $SiO_2$, eluting with the following gradient:

| | |
|---|---|
| 5% AcOEt - 95% n-heptane | 2.5 L |
| 10% AcOEt - 90% n-heptane | 2 L |
| 15% AcOEt - 85% n-heptane | 2 L |
| 20% AcOEt - 80% n-heptane | 5 L |

***(R,S)-4-Benzyl-morpholine-2-carboxylic acid ethyl ester (1a,1b)***

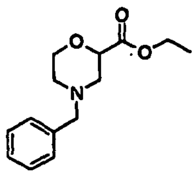

A stirred solution of 4-benzyl-morpholine-2-carbonitrile (113.0g, 0.56mole) in ethanol (1030ml) is treated with concentrated sulphuric acid (165ml) added in portions. (exothermic, internal temperature rises from ambient to 65 °C). The mixture is then warmed under reflux for 66hrs. The solution is cooled and then concentrated in vacuo to half volume, basified with aqueous potassium carbonate (beware frothing) and the product extracted into diethyl ether. The organic phase is dried over magnesium sulphate, filtered and evaporated to dryness in vacuo to yield an oil. This material is evacuated further under high vacuum. Yield = 121.3g (87%).

***(R,S)-4-Benzyl-morpholine-2-carboxylic acid methoxy-methyl-amide (2a, 2b)***

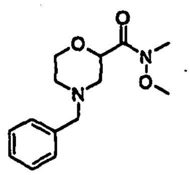

To a stirred suspension of *N,N*-dimethylhydroxylamine (6.6 g, 67.6 mmol) in anhydrous DCM (200 ml) under nitrogen at 0°C is added dropwise a solution of trimethylaluminium (2M solution in hexane, 34 ml, 67.6 mmol) over 30 minutes. The reaction mixture is allowed to warm up to room temperature and left stirring for 1 hour. A solution of the ester **1a, 1b** (6.74 g, 27 mmol) in anhydrous DCM (100 ml) is then added dropwise over 30 minutes and the reaction mixture left stirring overnight before quenching by cautious addition of phosphate buffer (disodium hydrogen phosphate, pH 8) solution. The precipitate is removed by filtration through a celite pad and the residue washed with chloroform. The organic phase is then concentrated *in vacuo* and washed with water. The aqueous layer is re-extracted with chloroform and the organic phases are combined, washed with brine, dried over magnesium sulphate and the solvent evaporated *in vacuo* to give **2a**, **2b** as a yellow oil. Alternatively, the reaction could be worked up as follows: upon addition of a solution of the ester **1a, 1b** (1 eq) the reaction mixture is left stirring for 1 hour before quenching by addition of phosphate buffer (disodium hydrogen phosphate, pH 8) solution, followed by addition of water. The aqueous layer is re-extracted with DCM and the organic phases are combined, dried over magnesium sulphate and the DCM evaporated *in vacuo* to give **2a, 2b** as a yellow oil (3.36 g, 47 %). MW 264.33; $C_{14}HN_2N_2O_3$; $^1H$ NMR ($CDCl_3$): 7.47-7.22 (5H, m), 4.55 (1H, d, 1.5 Hz), 4.00 (1H, dd, 11.5 Hz, 1.7 Hz), 3.75 (1H, dt, 11.5 Hz, 2.2 Hz), 3.65 (3H, s) 3.56 (2H, m), 3.17 (3H, s), 2.93 (1H, d, 11.3 Hz), 2.68 (1H, d, 11.3 Hz), 2.30 (2H, AB, 11.3 Hz); LCMS: (6 min method) m/z 265 $[M+H]^+$, Rt 0.65 min.

***2-Phenyl-5-fluoro benzyl bromide***

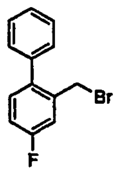

The title compound is prepared in 5 steps from commercially available (Aldrich) 5-fluorosalicylic acid following literature procedures (JACS, 2000, 122, 4020-4028). MW 265.13; $C_{13}H_{10}BrF$; $^1H$ NMR ($CDCl_3$): 7.48-7.38 (5H, m), 7.26-7.19 (1H, m), 7.05 (1H, td, 8.3 Hz, 2.8 Hz), 4.39 (2H, s); $^{19}F$ NMR ($CDCl_3$): -114.72.

***(5-Fluoro-2-methoxy-phenyl)-methanol***

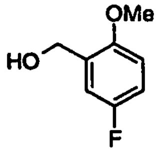

To a solution of 2-methoxy-5-fluorobenzaldehyde (11.093g, 1 equiv.- available from Aldrich Chemical Company) in methanol at -10 °C under nitrogen atmosphere is added $NaBH_4$ (7.515g, 2.7 equiv.) portionwise. The solution is allowed to warm to room temperature and after 30 minutes the reaction solvent is removed under reduced pressure and replaced with dichloromethane. This solution is poured onto ice water and further extracted with dichloromethane. The organic fractions are collected and dried ($MgSO_4$) and the solvent removed under reduced pressure to give the title compound as an oil (9.794g, 87%). $^1H$ NMR (300MHz, $CDCl_3$): $\delta$ 2.58 (m, 1H), 3.81 (s, 3H), 4.63 (d, 2H, *J* = 6.3 Hz), 6.78 (dd, 1H, *J* = 8.9 and 4.3 Hz), 6.94 (td, 1H, *J* = 8.5 and 3.1Hz), 7.04 (dd, 1H, *J* = 8.7 and 3.1Hz).

***5-fluoro-2-methoxybernzyl chloride***

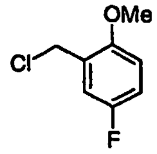

Neat (5-Fluoro-2-methoxy-phenyl)-methanol (19.587g, 1 equiv.) is added to neat $SOCl_2$ (42.2 mL, 4.6 equiv.) at -78°C under a nitrogen atmosphere and the solution is then allowed to warm to room temperature and stirred until evolution of gas ceases. An equivalent volume of anhydrous toluene is added to the flask and the solution heated to 60°C. On cooling, the reaction solution is poured onto ice water. The toluene layer is separated and dried ($MgSO_4$) and the solvent removed under reduced pressure. The crude material is sublimed (60-80°C/0.05 mBarr) to give the title compound as a white solid (13.40 g, 61%). $^1H$ NMR (300MHz, $CDCl_3$): $\delta$ 3.87 (s, 3H), 4.60 (s, 2H), 6.79-7.20 (m, 3H).

***2-methoxy-5-fluorobenzyl magnesium chloride***

Magnesium turnings (21.6 g, 0.888 mole, 2 eq.) and diethyl ether (300 ml) are loaded in a reactor under $N_2$. A solution of 5-fluoro-2-methoxybenzyl chloride (116 g, 0.664 mole, 1.5 eq.) in diethyl ether (200 ml) is loaded in an addition funnel. Iodine crystals and a small amount of the 5-fluoro-2-methoxybenzyl chloride solution are added and the reaction mixture is stirred to initiate the reaction. The remainder of the 5-fluoro-2 methoxybenzyl chloride solution is then added drop-wise maintaining the temperature of the reaction mixture below 28 °C. The mixture is stirred for another 5 minutes at 19 °C and after completion of the addition and a white suspension is formed.

***1-[4-(Phenylmethyl)morpholin-2-yl]ethan-1-one (3)***

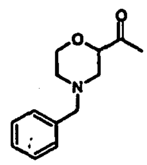

Compound **3** is obtained from **2b** (0.730 g, 2.8 mmol) and commercially available (Aldrich) methyl magnesium bromide (1.0M solution in THF, 3 ml, 3 mmol, 1.1 eq) in anhydrous THF (25 ml) following *General Procedure 1* after purification by automated column chromatography (EtOAc/n-heptane 14-100% gradient) (0.3 g, 49%). MW 235.33; $C_{14}H_{21}NO_2$. LCMS (6 minute method) m/z 220.1 $[M+H]^+$, $R_T$ 1.55 min.

***1-[4-(Phenylmethyl)morpholin-2-yl]propan-1-one (4)***

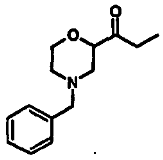

Compound **4** is obtained from **2b** (0.70 g, 2.65 mmol) and commercially available (Aldrich) ethyl magnesium bromide (2.65 ml, 7.94 mmol, 3 eq) in anhydrous THF (25 ml) following *General Procedure 1* as a yellow oil (583 mg,

89%). MW 249.36; $C_{15}H_{23}NO_2$.

*2-Methyl-1-[4-(phenylmethyl)morpholin-2-propan-1-one (5)*

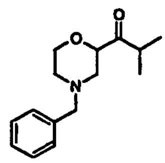

Compound **5** is obtained from **2b** (3.018 g, 11.4 mmol) and commercially available (Aldrich) isopropyl magnesium chloride (2M/THF, 17.1 ml, 34.3 mmol, 3 eq) in THF (100 ml), following *General Procedure 1* as a yellow oil (2.68 g, 89%); MW 263.38; $C_{16}H_{25}NO_2$); LCMS (6 minute method): m/z 248.2 $[M+H]^+$, $R_T$ 2.41 min.

*3-Methyl-1-[4-(phenylmethyl)morpholin-2-yl]butan-1-one (6)*

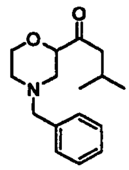

Compound **6** is prepared from **2** (10 g, 37 mmol) in anhydrous tetrahydrofuran (50 ml) and commercially available (Aldrich) isobutyl magnesium bromide (2M solution in diethyl ether, 56 mmol, 28 ml, 1.5 eq) following *General Procedure 1.* After stirring for 1 hour the reaction is quenched by addition of aqueous hydrochloric acid (150 ml). THF is removed in vacuo and diethyl ether is added after pH adjustment by addition of a saturated sodium bicarbonate solution. The organic phases are combined, dried over magnesium sulphate and the solvent is removed *in vacuo.* **6** is isolated in 80% purity (8.7 g, 67 % with respect to pure product). MW 261.37; $C_{16}H_{23}NO_2$; LCMS: (6 min method) m/z 262.2 $[M+H]^+$, $R_T$ 2.753 min.

*Cyclopentyl[4-(phenylmethyl)morpholin-2-yl]methanone (7)*

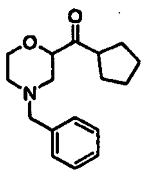

Compound **7** is prepared from **2** (3.36 g, 12.7 mmol) in anhydrous tetrahydrofuran (120 ml) and commercially available (Aldrich) cyclopentyl magnesium bromide (2M solution in diethyl ether, 19.1 ml, 38.2 mmol, 3 eq following *General Procedure* 1 in quantitative yield as yellow oil. MW 273.38; $C_{17}H_{23}NO_2$; LCMS: (6 min method) m/z 274 $[M+H]^+$, $R_T$ 2.24 min.

*[4-(Phenylmethyl)morpholin-2-yl](tetrahydro-2H-pyrna-4-yl)methanone (8)*

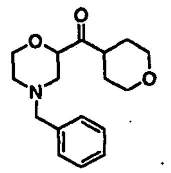

Compound **8** is obtained from **2b** (2.84 g, 10.74 mmol) in anhydrous tetrahydrofuran (30 ml) and 4-tetrahydropyranyl magnesium chloride (Chem. Ber. 98,1965, 3757) (2M solution in tetrahydrofuran, 6.5 ml, 13 mmol, 1.2 eq) following *General Procedure 1.* After 30 minutes further 4-tetrahydropyranyl magnesium chloride is added (2M solution in diethyl ether, 6.5 ml, 13 mmol, 1 eq.). After stirring for 2 hours the reaction mixture is quenched by addition of ammonium chloride solution (30 ml) and ethyl acetate (30 ml). The aqueous layer is re-extracted with ethylacetate.(30 ml) and the organic phases are combined, dried over magnesium sulphate and the solvents are removed *in vacuo.* The resulting residue is purified by ion exchange ion exchange chromatography to give **8** as a yellow oil (2.98 g, 96 %). MW 289.38; $C_{17}H_{23}NO_3$. LCMS: (6 min method) m/z 290 $[M+H]^+$, $R_T$ 2.20 min.

***5,5,5-Trifluoro-1-[4-(phenylmethyl)morpholin-2-yl]butan-1-one (9)***

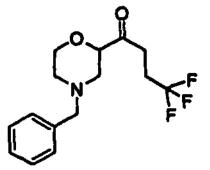

Compound **9** is obtained from **2b** (1.38g, 5.23mmol) and 3,3,3-trifluoropropyl magnesium bromide (20.9mls, 10.50mmol, 2eq) in dry THF (45ml) following *General procedure* 1. 3,3,3-Trifluoropropyl magnesium bromide is obtained from commercially available (Aldrich) 3,3,3-trifluoropropyl bromide following *General Procedure 5.* Purification by ion exchange chromatography gives **9** as oil (1.24g, 78.7%). MW 301.31; $C_{15}H_{18}F_3NO_2$LCMS (6 minute method): m/z 302.4 $[M+H]^+$, $R_T$ 2.66min.

***5,5,5-Trifluoro-1-[4-(phenylmethyl)morpholin-2-yl]pentan-1-one (10)***

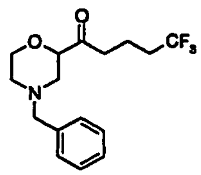

Compound **10** is prepared from a solution of **2** (0.717 g, 2.71 mmol) in anhydrous tetrahydrofuran (20 ml) and 4,4,4-trifluorobutyl magnesium bromide (0.5M solution in diethyl ether, 6.5 ml, 3.25 mmol, 1.2eq). 4,4,4-Trifluorobutyl magnesium bromide is obtained from commercially available (Aldrich) 4,4,4-trifluorobutyl bromide following *General Procedure 5.* After 30 minutes another 0.3 eq of 4,4,4-trifluorobutyl magnesium bromide are added (0.5M solution in diethyl ether, 2.5 ml). After stirring for 2 hours the solvents are removed *in vacuo* and water (20 ml) and ethyl acetate (30 ml) are added to the residue. The organic phase is washed with brine, dried over magnesium sulphate and the solvent is removed *in vacuo* to give **10** as clear oil (0.985 g). **10** is taken onto the next step without further purification. MW 315.34; $C_{16}H_{20}NO_2F_3$; LCMS: (6 min method) m/z 316 $[M+H]^+$, $R_T$ 2.9min.

***4-Benzylmorpholin-3-one***

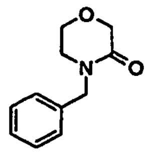

A solution of *N*-benzyl-*N*-(2-hydroxyethyl) chloroacetamide (627.7 g, 2.76 mol) in *tert*-butanol (0.91) is stirred under nitrogen while warming to 25-30°C. Potassium tert-butoxide (2.8971 of a 1M solution in *tert*-butanol, 2.90 mol, 1.05 eq) is added over 2 hours. The reaction mixture is then stirred at room temperature for 90 minutes. Ice-cold water (61) is added and the resultant cloudy solution extracted with ethyl acetate. The combined organic layers are washed with brine, dried over magnesium sulphate and evaporated *in vacuo* to give a light brown oil (441 g, 84%), which is used in the next stage without further purification; MW 191.23; $C_{11}H_{13}NO_2$; $^1H$ NMR ($CDCl_3$): 7.29-7.40 (5H, m), 4.67 (2H, s), 4.28 (2H, s), 3.87 (2H, t, 5 Hz), 3.31 (2H, t, 5 Hz); LCMS: (12 min method) m/z 192 [M+H]+ @ Rt 1.00 min.

***4-Benzyl-2-(cyclopropyl-hydroxy-methyl)morpholin-3-one (75)***

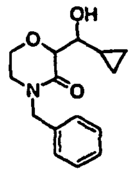

To a solution of 4-benzyl-morpholin-3-one (9.5 g, 50 mmol) in THF (200 ml) is added lithium diisopropylamide (2M solution in THF, 27 ml, 54 mmol, 1.1 eq) dropwise over 20 minutes at -78°C followed by slow addition of cyclopropyl methylaldehyde (3.85 ml, 55 mmol, 1.1 eq). After stirring at -78°C for one hour the reaction mixture is allowed to warm to room temperature and stirred for another 6 hours. The reaction is quenched by addition of EtOAc and brine. The aqueous layer is extracted with EtOAc, the combined organic layers are dried over magnesium sulphate and reduced *in vacuo.* Purification using automated column chromatography (DCM/MeOH, 0 to 15% gradient) gives 75 in 70% purity with 4-benzyl-morpholin-3-one as the major impurity. This product is directly used in the next step. MW 261.32; $C_{15}H_{19}NO_3$; $^1H$ NMR ($CDCl_3$): LCMS: (6 min method) m/z 261.32 $[M+H]^+$, $R_T$ 2.23

***(4-Benzyl-morpholin-2-yl)-cyclopropyl-methanol (76)***

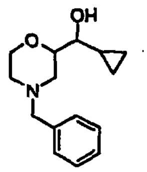

Borane-THF complex (1M solution in THF, 30 ml, 30 mmol, 4.1 eq) is added slowly to a solution of **75** (1.9 g, 7.3 mmol) in THF (100 ml). The reaction is heated to 60°C. After 24 hours MeOH and hydrochloric acid (2M, excess) are added and the resulting mixture heated for one hour at the same temperature. After careful addition of saturated $NaHCO_3$ solution and EtOAc the aqueous layer is extracted with EtOAc. The combined organic layers are washed with, brine, dried over magnesium sulphate and the solvent is removed *in vacuo.* Purification by ion exchange chromatography

gives **76** (1.1 g, 61%). MW 247.34; $C_{15}H_{21}NO_2$; $^1H$ NMR ($CDCl_3$): LCMS: (6 min method) m/z 0.64 $[M+H]^+$, $R_T$ 2.48 min.

***Cyclopropyl[4-(phenylmethyl)morpholin-2-yl]methanone (77)***

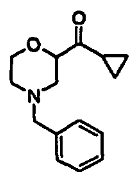

A solution of dimethylsulphoxide (0.69 ml, 9.7 mmol, 2.2 eq) in DCM (4.5 ml) is slowly added to a solution of oxalyl chloride (2.43 ml, 4.85 mmol, 1.1 eq) in DCM (2.5 ml) followed by a solution of **76** (1.09 g, 4.41 mmol) in DCM (0.7 ml) under nitrogen at - 60°C. After stirring for 15 minutes, triethylamine (3.14 ml, 22.1 mmol, 5 eq) is added and stirring continues for 15 minutes. After addition of water, the layers are separated. The aqueous layer is washed with DCM. The combined organic layers are washed with brine, dried over magnesium sulphate and the solvent is removed *in vacuo.* Purification using automated column chromatography (EtOAc/n-hexane, 20-50% gradient) gives **77** as a yellow oil (0.69 g, 64%). MW 245.32; $C_{15}H_{19}NO_2$; $^1H$ NMR ($CDCl_3$): LCMS: (6 min method) m/z 246.3 $[M+H]^+$, RT 1.095min.

**<u>Example 1H: Preparation of 1-[1,1'-biphenyl]-2-yl-2-morpholin-2-ylpropan-2-ol hydrochloride (12)</u>**

***1-[1,1'-Biphenyl]-2-yl-2-[4-(phenylmethyl)mopholin-2-ylpropan-2-ol (11)***

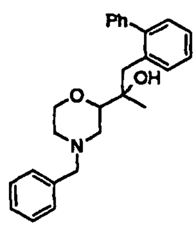

Compound **11** is prepared from 2-phenylbenzyl magnesium bromide (0.25M solution in diethyl ether, 5.5 ml, 1.38 mmol) and **3** (275 mg, 1.25 mmol) in anhydrous THF (7 ml) following *General Procedure* 2). 2-Phenylbenzyl magnesium bromide is obtained from commercially available (Aldrich) 2-phenylbenzyl bromide following *General Procedure* 5. Further equivalents of 2-phenylbenzyl magnesium bromide (10 ml, 2.5 mmol) are added before quenching the reaction with ice water (7 ml). **11** is obtained as an oil in 75% purity after ion exchange (5 g column) chromatography and automated column chromatography (0-50% EtOAc/heptane gradient) and taken onto the next step without further purification (0.23 mg isolated material). MW 387.53; $C_{26}H_{29}NO_2$. LCMS (6 minute method): m/z 388.2 $[M+H]^+$, $R_T$ 3.37 min.

***1-[1,1'-Biphenyl]-2-yl-2-mopholin-2-ylpropan-2-ol hydrochloride (12)***

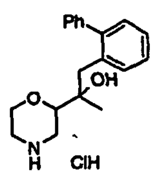

**12** is obtained from **11** (204 mg, 0.53 mmol), α-chloroethyl chloroformate (0.23 ml, 2.11 mmol) and polymer-supported Hünig's base (296 mg, 1.05 mmol) in DCM (5 ml) following *General Procedure 3.* Purification using ion exchange chromatography, followed by preparative LCMS and conversion into the hydrochloride salt following *General Procedure 4* gives **12** as a foam (102 mg, 65%). MW 297.36; $C_{19}H_{23}NO_2$. HCl; $^1$H NMR ($CD_3OD$) $\delta_H$ 7.15-7.39 (8H, m), 7.07-7.11 (1H, m), 3.97 (1H, dd, 3.0 Hz, 13.0 Hz), 3.56-3.65 (1H, m), 3.20-3.25 (1H, m), 3.08 (2H, t, 12.5 Hz), 2.82-2.99 (4H, m), 0.60 (3H, s). LCMS (12 minute method): m/z 298.2 $[M+H]^+$, $R_T$ 4.38 min.

**Example 2H: Preparation of 1-[5-fluoro-2-(methyloxy)phenyl]-2-morpholin-2-ylbutan-2-ol hydrochloride (14)**

***1-[5-Fluoro-2-(methyloxy)phenyl]-2-[4-(phenylmethyl)morpholin-2-yl]butan-2-ol (13)***

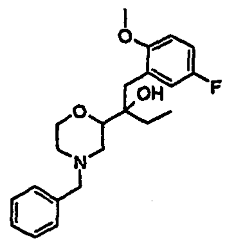

Compound **13** is obtained from **4** (583 mg, 2.5 mmol) and 2-methoxy-5-fluorobenzyl magnesium bromide (5.5 ml, 2.75 mmol, 1.1eq) in anhydrous THF (15 ml) following *General Procedure* 2. Further equivalents of 2-methoxy-5-fluorobenzyl magnesium bromide. (2M solution in diethyl ether, 10 ml, 5.0 mmol) are added after 30 min and the mixture is warmed to room temperature and left to stir over night. After purification by ion exchange chromatography **13** is obtained as a yellow oil in 67% purity (702 mg). The compound is taken over to the next step without further purification. MW 373.47, $C_{22}H_{28}FNO_3$. LCMS (6 minute method) m/z 374.2 $[M+H]^+$, $R_T$ 3.17 min.

***1-[5-Fluoro-2-(methyloxy)phenyl]-2-morpholin-2-ylbutan-2-ol hydrochloride (14)***

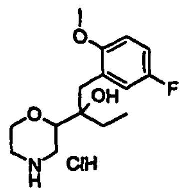

**14** is obtained from **13** (717 mg, 1.92 mmol), α-chloroethyl chloroformate (0.83 ml, 3.84 mmol, 4eq) and polymer-supported Hünig's base (1.08 g, 3.84 mmol, 2eq) in DCM (17 ml) following *General Procedure 3.* Purification by ion exchange chromatography followed by preparative LCMS and conversion into the hydrochloride salt following *General Procedure 4* gives **14** as a solid (185 mg, 30%). MW 319.81; $C_{15}H_{22}FNO_3$.HCl. $^1$H NMR ($CD_3OD$) $\delta_H$ 6.94 (1H, dt, 1.5 Hz, 9 Hz), 6.81-6.84 (2H, m), 4.07 (1H, dd, 3.5, 13 Hz), 3.67-3.76 (4H, m), 3.56 (1H, dd, 2.5 Hz, 11 Hz), 3.33 (1H, m), 3.14-3.25 (1H, m), 3.00-3.08 (2H, m), 2.84 (2H, AB; 14 Hz), 1.37-1.51 (1H, m), 1.05-1.19 (1H, m), 0.82 (3H, t, 7.5 Hz). LCMS (12 minute method): m/z 284.1 $[M-HCl+H]^+$, $R_T$ 3.76 min.

**Example 3H: Preparation of 2-morpholin-2-yl-1-{2-[(trifluoromethyl)oxy] phenyl}butan-2-ol hydrochloride (16)**

***2-[4-(phenylmethyl)morpholin-2-yl]-1-{2-[trifluoromethyl)oxy]phenyl}butan-2-ol (15)***

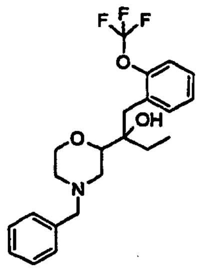

Compound **15** is obtained from **4** (1.1 mg, 4.71 mmol) and commercially available (Fluorochem) 2-trifluoromethoxy benzyl magnesium bromide (10.4 ml, 5.19 mmol, 1.1eq) in anhydrous THF (31 ml) following *General Procedure 2.* After 30 minutes further equivalents of 2-trifluoromethoxy benzyl magnesium bromide are added (0.5M solution in diethyl ether, 4.71 ml, 2.36 mmol). Purification by ion exchange chromatography gives **15** as an oil (1.88 g, 98%). MW 409.45; $C_{22}H_{26}F_3NO_3$. LCMS (6 minute method): m/z 410.4 $[M+H]^+$, $R_T$ 3.28 min.

***2-Morpholin-2-yl-1-{2-[(trifluoroinetloyl)oxy]phenyl}butan-2-ol hydrochloride (16)***

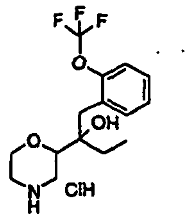

**16** is obtained from **15** (1.88 g, 4.59 mmol), α-chloroethyl chloroformate (1.98 ml, 18.4 mmol) and polymer-supported Hünig's base (2.58 g, 9.18 mmol) in DCM (40 ml) following *General Procedure* 3. Purification using ion exchange chromatography followed by automated column chromatography (0-20% MeOH/DCM gradient) and conversion to the hydrochloride salt following *General Procedure 4* gives **16** (258.5 mg, 17%) as a white solid. MW 319.33, $C_{15}H_{20}F_3NO_3$.HCl. $^1$H NMR ($CD_3OD$) $\delta_H$ 7.53 (1H, dd, 2 Hz, 7.5 Hz), 7.27-7.38 (3H, m), 4.20 (1H, dd, 3.5 Hz, 13 Hz), 3.85 (1H, td, 3 Hz, 13 Hz), 3.70 (1H, dd, 2 Hz, 11 Hz), 3.44 (1H, d, 13 Hz), 3.27-3.34 (1H, m), 3.12-3.22 (2H, m), 3.07 (1H, d, 14 Hz), 2.96 (1H, d, 14 Hz), 1.55 (1H, sextet, 7.5 Hz), 1.26 (1H, sextet, 7.5 Hz), 0.93 (3H, t, 7.5 Hz). LCMS (12 minute method): m/z 320.4 $[M+H]^+$, $R_T$ 2.77 min.

**Example 4H: Preparation of 1-[1,1'-biphenyl]-2-yl-2-morpholin-2-ylbutan-2-ol hydrochloride (18)**

***1-[1,1'-Biphenyl]-2-yl-[4-(phenylmethyl)morpholin-2-ylbutan-2-ol (17)***

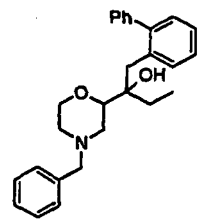

Compound **17** is obtained from **3** (601 mg, 2.58 mmol) and 2-phenylbenzyl magnesium bromide (0.25M solution in diethyl ether, 11.5 ml, 2.84 mmol) in anhydrous THF (15 ml) following *General Procedure* 2. 2-Phenylbenzyl magnesium bromide is prepared from commercially available (Aldrich) 2-phenylbenzyl bromide following *General Procedure* 5. Further equivalents of 2-phenylbenzyl magnesium bromide (10.32 ml, 2.58 mmol) are added. Purification by ion exchange

chromatography followed by automated column chromatography (0-50% EtOAc/n-heptane, gradient) gives **17** (705 mg, 68%) as a colourless oil in 91 % purity which is directly used in the next step. MW 401.55, $C_{27}H_{31}NO_2$. LCMS (6 minute method): m/z 402.2 $[M+H]^+$, $R_T$ 3.56 min

***1-[1,1'-Biphenyl]-2-yl-2-morpholin-2-ylbutan-2-ol hydrochloride (18)***

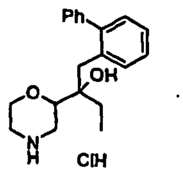

**18** is obtained from **17** (705 mg, 1.76 mmol), α-chloroethyl chloroformate (0.76 ml, 7.02 mmol) and polymer-supported Hünig's base (988 g, 3.52 mmol) in DCM (15 ml) following *General Procedure* 3. Purification by ion exchange chromatography followed by automated column chromatography (5-20% MeOH/DCM gradient) and conversion into the hydrochloride salt following *General Procedure 4* gives **18** (0.37 g, 62%) as a yellow foam. MW 347.82, $C_{20}H_{25}NO_2$.HCl $^1H$ NMR ($CD_3OD$) $\delta_H$ 7.43-7.46 (1H, m), 7.29-7.34 (3H, m), 7.14-7.25 (5H, m), 7.06-7.11 (1H, m), 3.94 (1H, dd, 3.5 Hz, 13 Hz), 3.55-3.64 (1H, m), 3.37 (1H, dd, 1.5 Hz, 11 Hz), 3.09 (2H, d, 12.5 Hz), 2.80-2.99 (4H, m), 1.11-1.23 (1H, m), 0.91 (1H, m), 0.38 (3H, t, 7.5 Hz). LCMS (12 minute method): m/z 312.1 $[M+H]^+$, $R_T$ 4.67 min.

**Example 5H: Preparation of 1-[5-fluoro-2-(methyloxy)phenyl]-3-methyl-2-morpholin-2-ylbutan-2-ol hydrochloride (20)**

***1-[5-Fluoro-2-(methoxy)phenyl]-3-methyl-2-[4-(phenylmethyl)morpholin-2-yl]butan-2-ol (19)***

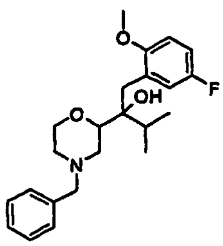

Compound **19** is obtained from **5** (0.7 g, 2.83 mmol) and 2-methoxy-5-fluoro-benzyl magnesium bromide (6.2 ml, 3.11 mmol, 1.1eq) in anhydrous THF (15 ml) following *General Procedure 2*. Further equivalents of 2-methoxy-5-fluoro-benzyl magnesium bromide (8.49 ml, 4.25 mmol) are added and mixture is warmed to room temperature and left to stir over night. Purification using automated column chromatography (0-25% n-heptane/EtOAc gradient) gives **19** (0.53 g, 48%). MW 387.5; $C_{23}H_{30}FNO_3$. LCMS (6 minute method): m/z 388.2 $[M+H]^+$, $R_T$3.21 min.

***1-[5-fluoro-2-(methyloxy)plzenyl]-3-methyl-2-morpholin-2-ylbutan-2-hydrochloride (20)***

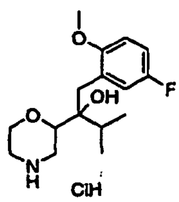

**20** is obtained from **19** (523 mg, 1.35 mmol), α-chloroethyl chloroformate (0.58 ml, 5.40 mmol, 4eq) and PS-DIEA (0.76 g, 2.70 mmol, 2eq) in DCM (10 ml) following *General Procedure 4.* Purification by ion exchange chromatography and conversion to the hydrochloride salt following *General Procedure 4* gives **20** as an off-white solid (0.26g, 58%). MW 333.83, $C_{16}H_{24}FNO_3$. HCl. $^1$H NMR ($CD_3OD$) $\delta_H$ 7.10 (1H, d, 9.5 Hz), 6.94 (2H, d, 6 Hz), 4.07 (1H, dd, 3.5 Hz, 13 Hz), 3.71-3.88 (5H, m), 3.21-3.47 (2H, m), 2.99-3.11 (4H, m), 1.8 (1H, septet, 7 Hz), 1.04 (3H, d, 7 Hz), 0.94 (3H, d, 7.0 Hz). LCMS (12 minute method): m/z 298 $[M-HCl+H]^+$, $R_T$ 4.29 min.

**Example 6H: Preparation of 3-methyl-1-[2-methyloxy)phenyl]-2-morpholin-2-ylbutan-2-ol hydrochloride (22a, 22b)**

***2-(4-Benzyl-morpholin-2-yl)-1-(2-methoxy-phenyl)-3-methyl-butan-2-ol (21)***

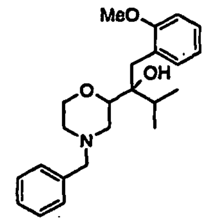

Compound **21** is obtained from **5** (1.5 g, 6.06 mmol) and 2-methoxy benzyl magnesium bromide (available from Rieke-Metals) (0.25M solution in THF, 33.9 ml, 8.49 mmol) in anhydrous THF (30 ml) following *General Procedure 1*. Purification by column chromatography (0-40% EtOAc/n-heptane gradient) gives **21** as colourless oil (1.45 g, 84%). MW 369.51, $C_{23}H_{31}NO_3$.HCl. LCMS (6 minute method): m/z 370.2 $[M+H]^+$, $R_T$ 2.77 min.

***3-Methyl-1-[2-methyloxy)phenyl]-2-morpholin-2-ylbutan-2-ol hydrochloride (22a, 22b)***

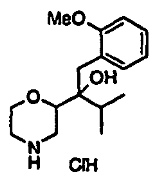

**22a,22b** is obtained from **21** (1.24 mg, 3.37 mmol), α-chloroethyl chloroformate (3.63 ml, 33.7 mmol) and polymer-supported Hünig's base (4.72 g, 16.8 mmol) in DCM (45 ml) following *General Procedure* 3. Purification using ion exchange chromatography followed by chiral preparative HPLC (Heptane:EtOH:DEA 85:15:0.2 gradient, chiralcel-OD) gives the first eluting enantiomer **22a** (RT 9.5min), and the second eluting enantiomer **22b** (RT 11.41min). The two enantiomers are converted to their respective hydrochloride salt **22a** (146 mg) and **22b** (138 mg) and obtained as white solids (28% overall combined yield). MW 315.84; $C_{16}H_{25}NO_3$.HCl. $^1$H NMR ($CD_3OD$) $\delta_H$ 7.22-7.34 (2H, m), 6.85-6.95 (2H, m), 4.08 (1H, dd, 3.6 12.8 Hz), 3.86-3.9 (4H, m), 3.77 (1H, td, 2.45 Hz, 12.4 Hz), 3.22-3.28 (1H, m), 3.24 (1H, d, 12.8 Hz), 2.95-3.11 (4H, m), 1.83 (1H, septet, 6.8 Hz), 1.16 (3H, d, 7.0 Hz), 0.95 (3H, d, 7.0 Hz). LCMS (12 minute method): m/z 280.2 $[M-HCl+H]^+$, $R_T$ 4.05 min.

**Example 7H: Preparation of 1-[2-ethyloxy)phenyl]-3-methyl-2-morpholin-2-ylbutan-2-ol hydrochloride (24a, 24b)**

***1-[2-Ethyloxy)phenyl]-3-methyl-2-[4-phenylmethyl)morpholin-2-ylbutan-2-ol (23)***

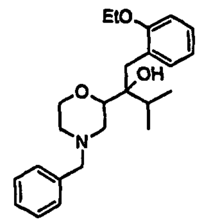

Compound **23** is obtained from **5** (1.5 g, 6.06 mmol) and 2-ethoxybenzyl magnesium chloride (available from Rieke-Metals) (0.25M solution in THF, 34 ml, 8.49 mmol) in anhydrous THF (30 ml) following *General Procedure* 2. After repeated purification by column chromatography (100% DCM to 10% MeOH/ DCM gradient followed by 100% DCM to 1:1 EtOAc: DCM gradient) **23** is obtained as colourless oil (0.8 g, 35%). MW 383.54, $C_{24}H_{33}NO_3$. LCMS (6 minute method): m/z 384.4 $[M+H]^+$, $R_T$ 3.04 min.

***1-[2-Ethyloxy)phenyl]-3-methyl-2-morpliolin-2-ylbutan-2-ol hydrochloride (24a, 24b)***

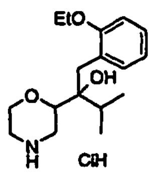

**24a, 24b** are obtained from **23** (766 mg, 2.0 mmol), α-chloroethyl chloroformate (0.86 ml, 8.0 mmol) and polymer-supported Hünig's base (1.12 g, 4.0 mmol) in DCM (30 ml) following *General Procedure* 3. Purification using ion exchange chromatography followed by automated column chromatography (0-20% MeOH/ DCM gradient) and chiral preparative chromatography (Heptane:EtOH:DEA 95:5:0.2 gradient, chiracel AD) gives the first eluting enantiomer, $R_T$ 13.40min, and the second eluting enantiomer, $R_T$ 15.63min. After conversion to their respective hydrochloride salt **24a** (85 mg) and **24b** (79 mg) are obtained as brown solids (28% combined yield). MW 293.36; $C_{17}H_{27}NO_3$.HCl. $^1H$ NMR ($CD_3OD$) $\delta_H$ 7.06-7.09 (1H, m), 6.95-7.01 (1H, m), 6.66-6.75 (2H, m), 3.80-3.92 (3H, m), 3.46-3.63 (2H, m), 2.96-3.15 (2H, m), 2.66-2.86 (4H, m), 1.54-1.63 (1H, m), 1.22 (3H, t, 7.0 Hz), 0.82 (3H, d, 7.0 Hz), 0.71 (3H, d, 7.0 Hz). LCMS (12 minute method): m/z 294.2 $[M+H]^+$, $R_T$4.60 min.

**<u>Example 8H: 3-Methyl-2-morpholin-2-yl-1-{2-[(trifluoromethyl)oxy]butan-2-ol hydrochloride (26)</u>**

***3-Methyl-2-[4-(phenylmethyl)morpholin-2-yl]-1-{2-[(trifluoromethyl)oxy]phenyl}butan-2-ol (25)***

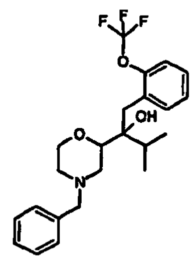

Compound **25** is obtained from **5** (953 mg, 3.85 mmol) and commercially available (Fluorochem) 2-trifluoromethoxy benzyl magnesium bromide (8.48 ml, 4.24 mmol, 1.1eq) in anhydrous THF (25 ml) following *General Procedure 3* and addition of further 2-trifluoromethoxy benzyl magnesium bromide (3.85 ml, 1.93 mmol). Purification by ion exchange chromatography gives **25** as a yellow oil in 86% purity which is used in the next step without further purification (1.53 g of isolated material). MW 423.38; $C_{23}H_{28}F_3NO_3$. LCMS (6 minute method) m/z 424.1 $[M+H]^+$, $R_T$ 3.53min.

*3-Methyl-2-moyholin-2-yl-1-{2-[(trifluoromethyl)oxy]butan-2-ol hydrochloride (26)*

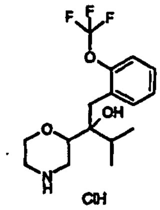

**26** is obtained from **25** (1.53 g, 3.61 mmol), α-chloroethyl chloroformate (1.55 ml, 14.5 mmol, 4eq) and polymer-supported Hünig's base (2.03 g, 7.23 mmol, 2eq) in DCM (30 ml) following *General Procedure 3*. Purification by ion exchange chromatography, followed by automated column chromatography (0-20% MeOH/DCM gradient) and conversion to its hydrochloride salt following *General Procedure 4* gives **26** as a yellow solid (0.4 g, 29%). MW 379.82; $C_{16}H_{22}F_3NO_3$.HCl. $^1$H NMR ($CD_3OD$) $\delta_H$ 7.46 (1H, dd, 1.5 Hz, 7.5 Hz), 7.14-7.24 (3H, m), 3.94 (1H, dd, 3.5 Hz, 13 Hz), 3.80 (1H, dd, 2.5 Hz, 11.5 Hz) 3.69 (1H, td, 2.5 Hz, 13 Hz), 3.27 (1H, d, 13 Hz), 3.13 (1H, d, 12.5 Hz), 2.72-3.02 (4H, m), 1.70 (1H, septet, 7 Hz), 0.94 (3H, d, 7 Hz), 0.84 (3H, d, 7.0 Hz). LCMS (12 minute method): m/z 334.4 $[M+H]^+$, $R_T$ 2.94 min.

<u>**Example 9H: Preparation of 1-[1,1'-biphenyl]-2-yl-3-methyl-2-morpholin-2-ylbutan-2-ol hydrochloride (28)**</u>

*1-[1,1'-Biphenyl]-2-yl-3-methyl-2-[4-(phenylmethyl)morpholin-2-ylbutan-2-ol (27)*

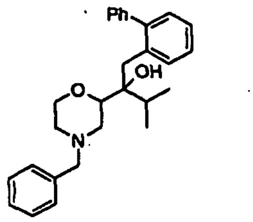

Compound **27** is obtained from **5** (0.7 g, 2.83 mmol) and 2-phenylbenzyl magnesium bromide (12.5 ml, 3.11 mmol) in anhydrous THF (15 ml) following *General Procedure 2* and further equivalents of 2-phenylbenzyl magnesium bromide reagent (11.3 ml, 5.66 mmol). 2-Phenylbenzyl magnesium bromide is prepared from commercially available (Aldrich) 2-phenylbenzyl bromide following *General Procedure 5.* Purification using ion exchange chromatography, followed by automated column chromatography (0-20% EtOAc/n-heptane gradient) gives **27** as oil (0.46 g, 40%). MW 415.58; $C_{28}H_{33}NO2$. LCMS (6 minute method): m/z 416.2 $[M+H]^+$, $R_T$3.45min.

*1-[1,1'-Biphenyl]-2-yl-3-methyl-2-morpholin-2-ylbutan-2-ol hydrochloride (28)*

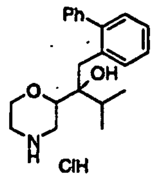

**28** is obtained from **27** (405 mg, 0.976 mmol), α-chloroethyl chloroformate (0.42 ml, 3.9 mmol) and polymer-supported Hünig's base (0.55 g, 1.95 mmol) in DCM (7 ml) following *General Procedure 3*. The crude product is purified

using ion exchange chromatography, and then converted to its hydrochloride salt following *General Procedure 4* to give **28** as a white solid (0.23 g, 71%). MW 361.91; $C_{21}H_{27}NO_2$.HCl. $^1$H NMR ($CD_3OD$) $\delta_H$ 7.61-7.64 (1H, m), 7.19-7.47 (8H, m), 3.95 (1H, dd, 4 Hz, 13 Hz), 3.61-3.71 (2H, m), 3.04- 3.19 (4H, m), 2.96 (1H, td, 4 Hz, 12.6 Hz), 2.70 (1H, dd, 13, 11.5 Hz), 1.67 (1H, septet, 7 Hz), 0.75 (3H, d, 7 Hz), 0.63 (3H, d, 7 Hz). LCMS (12 minute method): m/z 326.2 $[M+H]^+$, $R_T$5.02 min.

**<u>Example 10H: Preparation of 1-(4-Fluoro[1.1'-biphenyl]-2-yl)-3-methyl-2-moripholin-2-ylbutan-2-ol hydrochloride (30)</u>**

***1-(4-Fluoro[1,1'-biphenyl]- 2-yl)- 3-methyl- 2-[4-(phenylmethyl) morpholin- 2-ylbutan- 2-ol hydrochloride (29)***

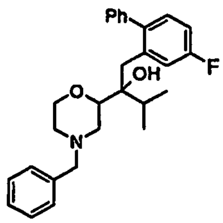

Compound **29** is obtained from **5** (1.13 g, 4.57 mmol) and 2-phenyl-5-fluorobenzyl magnesium bromide (0.5M in THF, 10.5 ml, 5.03 mmol) in anhydrous THF (30 ml) following *General Procedure 2* (further 2-phenyl-5-fluorobenzyl magnesium bromide (0.33eq, 3 ml, 1.51 mmol) is added after 30 min). 2-Phenyl-5-fluorobenzyl magnesium bromide is obtained from 2-phenyl-5-fluorobenzyl bromide following *General Procedure* 5. Purification by ion exchange chromatography followed by automated column chromatography (0-20% EtOAc/n-heptane gradient) gives **29** as a yellow oil in 86% purity which is directly used in the next step (1.58 g recovered material). MW 415.58; $C_{28}H_{33}NO_2$. LCMS (6 minute method): m/z 434.5 $[M+H]^+$, $R_T$3.71 min

***1-(4-Fluoro[1,1'-biphenyl]-2-yl)-3-methyl-2-morpholin-2-ylbutan-2-ol hydrochloride (30)***

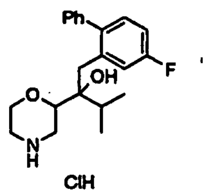

**30** is obtained from **29** (1.58 g, 3.63 mmol), α-chloroethyl chloroformate (1.57 ml, 3.63 mmol) and polymer-supported Hünig's base (2.04 g, 7.26 mmol) in DCM (30 ml) following *General Procedure 3*. The crude product is purified using ion exchange chromatography, automated column chromatography (0-20% MeOH/ DCM gradient, 40 g column), and preparative LCMS. Conversion to the hydrochloride salt following *General Procedure 4* gives **30** as a yellow solid (0.3 g, 22%). MW 379.91; $C_{21}H_{26}FNO_2$.HCl. $^1$H NMR ($CD_3OD$) $\delta_H$ 7.20-7.35 (6H, m), 7.07-7.14 (1H, m), 6.90 (1H, td, 2.5 Hz, 8.5 Hz), 3.83 (1H, d, br, 10 Hz), 3.56 (2H, t, 10 Hz), 3.03-3.12 (2H, m), 2.79-2.98 (3H, m), 2.63 (1H, t, 11.5 Hz), 1.55 (1H, quintet, 7 Hz), 0.64 (3H, d, 7 Hz), 0.51 (3H, d, 7 Hz). LCMS (12 minute method): m/z 344.1 $[M-HCl+H]^+$, $R_T$5.14 min.

**<u>Example 11H: Preparation of 1-[5-fluoro-2-(methyloxy)phenyl]-4-methyl-2-morpholin-2-yl-pentan-2-ol hydrochloride (32)</u>**

***1-[5-Fluoro-2-(methyloxy)phenyl]-4-methyl-2-[4-(phenylmethyl)morpholin-2-yl]pentan-2-ol (31)***

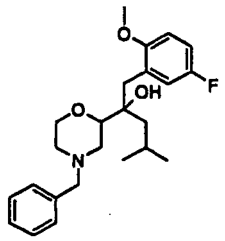

Compound **31** is obtained from **6** (465 mg, 1.78 mmol) and 2-methoxy-5-fluorobenzyl magnesium bromide (3.92 ml, 1.96 mmol, 1.1eq) in dry THF (10 ml) following *General Procedure 2*. Purification by ion exchange chromatography followed by automated column chromatography (0-40% EtOAc/n-heptane gradient) gives **31** as an oil (448 mg, 83% purity). MW 401.53; $C_{24}H_{32}FNO_3$. LCMS (6 minute method): m/z 402.2 $[M+H]^+$, $R_T$ 3.40 min.

***1-[5-Fluoro-2-(methyloxy)phenyl]-4-methyl-2-morpholin-2-ylpefuan-2-ol hydrochloride (32)***

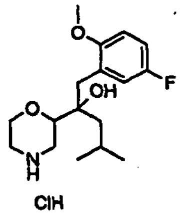

**32** is obtained from **31** (448 mg, 1.12 mmol), α-chloroethyl chloroformate (0.48 ml, 4.47 mmol, 4eq) and polymer-supported Hünig's base (628 g, 2.23 mmol, 2eq) in DCM (10 ml) following *General Procedure 3*. Purification by ion exchange chromatography followed by preparative LCMS and conversion to its hydrochloride salt following *General Procedure 4* gives **32** as a white solid (0.11 g, 32%). MW 347.72; $C_{17}H_{26}FNO_3$. HCl. $^1H$ NMR ($CD_3OD$) $\delta_H$ 7.05-7.08 (1H, m), 6.95-6.98 (2H, m), 4.16 (1H, dd, 3 Hz, 12.5 Hz), 3.75- 3.86 (4H, m), 3.67(1H, d, 10.5 Hz), 3.51 (1H, d, 12 Hz), 3.25-3.29 (1H, m), 3.07-3.20 (2H, m), 2.94 (2H, AB, 14 Hz), 1.86-1.9 (1H, m), 1.53 (1H, dd, 5.5 Hz, 14.5 Hz), 1.13 (1H, dd, 14.5 Hz, 5.5 Hz), 0.94 (3H, d, 2.5 Hz), 0.92 (3H, d, 2.5 Hz). LCMS (12 minute method): m/z 312.1 $[M-HCl+H]^+$, $R_T$ 4.61 min.

**Example 12H: Preparation of 1-[2-(ethyloxy)phenyl]-4-methyl-2-morpholin-2-yipentan-2-ol 34a, 34b)**

***1-[2-(Ethyloxy)phenyl]-4-methyl-2-[4-(phenylmethyl)morpholin-2-ylpentan-2-ol (33)***

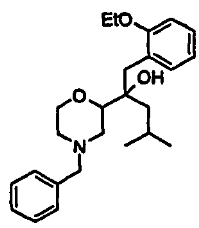

Compound **33** is obtained from **6** (3.0 g, 11.5 mmol) and 2-ethoxybenzyl magnesium chloride (available from Reike Metals) (0.25M in diethyl ether, 50.5 ml, 12.6 mmol) in anhydrous THF (55 ml) following *General Procedure 2*. Another two equivalents of 2-ethoxybenzyl magnesium chloride (92 ml, 23 mmol) are added after 30 min. Purification by automated column chromatography (0-25% EtOAc/n-heptane gradient) gives **33** (3.21 g) as a colourless oil in 86% purity as a mixture of diastereomers. MW 397.56; $C_{25}H_{35}NO_3$. LCMS (6 minute method): m/z 398.3 $[M+H]^+$, $R_T$ 3.42 & 3.60 min.

*1-[2-(Ethyloxy)phenyl]-4-methyl-2-morpholin-2-ylpentan-2-ol (34a, 34b)*

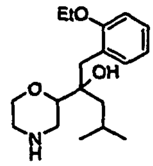

**34a, 34b** is obtained from **33** (3.20 mg, 8.06 mmol), α-chloroethyl chloroformate (3.48 ml, 32.2 mmol) and polymer-supported Hünig's base (4.53 g, 16.1 mmol) in DCM (100 ml) following *General Procedure 3.* Purification by ion exchange chromatography followed by automated column chromatography (5-40% MeOH/ DCM gradient), and preparative LCMS (gradient) gives **34a, 34b**. Chiral preparative chromatography (Heptane:EtOH:DEA 60:40:0.2 gradient, chiralcel-OD) afforded the first eluting enantiomer **34a** (13 mg) (RT 8.25 min), and the second eluting enantiomer **34b** (RT 10.17 min) as colourless oils. MW 307.44; $C_{18}H_{29}NO_3$. $^1H$ NMR ($CDCl_3$) $\delta_H$ 7.16-7.22 (2H, m), 6.84-6.93 (2H, m), 3.97-4.17 (2H, m), 3.90 (1H, dd, 3 Hz, 11 Hz), 3.53 (1H, td, 3 Hz, 11 Hz), 3.37 (1H, dd, 2 Hz, 10 Hz), 3.18 (1H, d, 12 Hz), 3.04 (1H, d, 14 Hz), 2.74-2.91 (4H, m), 1.89 (1H, septet, 6 Hz), 1.52 (1H, dd, 5.5 Hz, 14 Hz), 1.44 (3H, t, 7 Hz), 1.11 (1H, dd, 6 Hz, 14 Hz), 0.93 (3H, d, 7 Hz), 0.90 (3H, d, 7 Hz). LCMS (12 minute method): m/z 308.2 $[M-HCl+H]^+$, $R_T$ 4.92 min.

**Example 13H: Preparation of 4-methyl-2-morpholin-2-yl-1-{2[trifluoromethyl) oxy]phenyl}pentan-2-ol hydrochloride (36)**

*4-Methyl-2-[4-(phenylmethyl)morpholin-2-yl]-1-2[trifluoromethyl)oxy]phenyl}pentan-2-ol (35)*

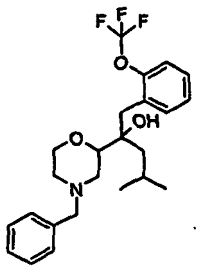

Compound **35** is prepared from **6** (0.83 g, 3.19 mmol) and commercially available (Fluorochem) 2-trifluoromethoxy benzyl magnesium bromide (0.5M solution in THF, 7.02 ml, 3.51 mmol, 1.1eq) in anhydrous THF (21 ml) following *General Procedure 2.* Further equivalents (3.19 ml, 1.60 mmol) of 2-trifluoromethoxy benzyl magnesium bromide are added after 30 min. Purification by ion exchange chromatography gives 35 as yellow oil (1.39 g, 99.5%). MW 437.51; $C_{24}H_{30}F_3NO_3$. LCMS (6 minute method): m/z 438.1 $[M+H]^+$, $R_T$3.70 min.

*4-Methyl-2-morpholin-2-yl-1-{2[trifluoromethyl)oxy]phenyl}pentan-2-ol hydrochloride (36)*

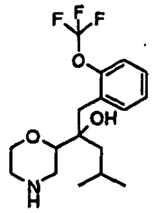

ClH

**36** is obtained from **35** (1.39 g, 3.18 mmol), α-chloroethyl chloroformate (1.37 ml, 12.7 mmol, 4eq), and polymer-supported Hünig's base (1.79 g, 6.36 mmol, 2eq) in DCM (25 ml) following *General Procedure 3*. Purification by ion exchange chromatography followed by preparative LCMS (gradient) and conversion into the hydrochloride salt following *General Procedure 4* gives **36** (0.16 g, 14%) as foam. MW 383.82; $C_{17}H_{24}F_3NO_3$.HCl. $^1$H NMR ($CD_3OD$) $\delta_H$ 7.43 (1H, d, 7 Hz), 7.16-7.27 (3H, m), 4.05 (1H, dd, 3 Hz, 13 Hz), 3.58-3.74 (2H, m), 3.35-3.40 (1H, m), 3.23- 3.14 (1H, m), 2.97-3.10 (3H, m), 2.76 (1H, d, 14 Hz), 1.75 (1H, septet, 6.5 Hz), 1.42 (1H, dd, 6 Hz, 14.5 Hz), 0.98-1.11 (1H, m) 0.83 (3H, d, 6 Hz), 0.81 (3H, d, 6 Hz). LCMS (12 minute method): m/z 348.4 $[M-HC1+H]^+$, $R_T$3.15 min.

**Example 14H: Preparation of 1-[1,1'-Biphenyl]-2-yl-4-methyl-2-moruholin-2-ylpentan-2-ol hydrochloride (38)**

***2-(4-Benzyl-morpholin-2-yl)-1-biphenyl-2-yl-4-methyl-pentan-2-ol (37a, 37b)***

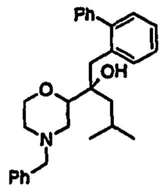

Compound **37** is prepared from **6** (2.5 g, 9.56 mmol) and 2-phenylbenzyl magnesium bromide (0.25M sol., 42.1 ml, 10.5 mmol, 1.1 eq) in anhydrous THF (21 ml) following *General Procedure 2*. 2-Phenylbenzyl magnesium bromide is prepared from commercially available (Aldrich) 2-phenylbenzyl bromide following *General Procedure 5*. Another 3 equivalents of 2-phenylbenzyl magnesium bromide are added to drive the reaction to completion. Purification by automated column chromatography (0-25%, EtOAc/n-heptane, gradient) gives **37** (2.07g, 50%) which is used in the next step without further purification. MW 429.61; $C_{29}H_{35}NO_2$. FIA: m/z 430 $[M+H]^+$.

***1-[1,1'-Biphenyl]-2-yl-4-methyl 2-morpholin-2-yl-pentan-2-ol (38a, 38b)***

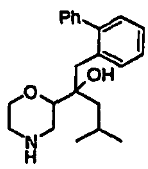

Compound **38** is obtained from **37** (2.07 g, 4.81 mmol), α-chloroethyl chloroformate (2.08 ml, 19.3 mmol) and polymer-supported Hünig's base (2.7 g, 9.6 mmol) in DCM (60 ml) following *General Procedure 3*. Purification by ion exchange chromatography, crystallization from MeOH/diethyl ether gives **38** as a white solid (738 mg, 45%). MW 339.48; $C_{22}H_{29}NO_2$. $^1$H NMR ($CDCl_3$) $\delta_H$ 7.29-7.46 (8H, m), 7.23-7.28 (1H, m), 3.79-3.91 (2H, m), 3.66 (1H, dd, 10.9 Hz, 1.7 Hz), 3.18 (2H, dd, 12.8 Hz, 25.8 Hz), 2.84-3.04 (3H, m), 2.75 (1H, t, 11.5 Hz), 1.56-1.68 (1H, m), 1.22 (1H, dd, 5.65 Hz, 14.7 Hz), 0.98 (1H, dd, 5.65 Hz, 14.7 Hz), 0.81 (3H, d, 3.2 Hz), 0.78 (3H, d, 3.0 Hz). LCMS (12 minute method): m/z 340.3 $[M+H]^+$, $R_T$ 5.62min.

***Phenylmethyl-2-[1,1'-biplienyl]-2-ylmethyl)-1-hydroxy-3-methylbutyl] morpholine-4-carboxylate (cbz-38a, cbz-38b)***

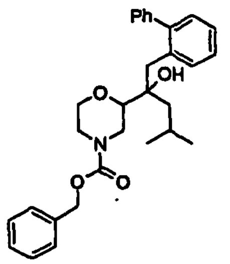

Benzyl chloroformate (0.37 ml, 2.61 mmol) is added to a stirring mixture of **38a,38b** (738 mg, 2.17 mmol) with $NaHCO_3$ (0.41 g) in a suspension of diethylether and water (24 ml) under $N_2$ at room temperature. After 1hour the reaction is quenched with ice water (15 ml) and diluted with DCM. The two phases are separated, the aqueous phase is further extracted DCM, the combined organic fractions are dried over magnesium sulphate, filtered and evaporated *in vacuo.* The isolated oil is purified using automated column chromatography (0-30% EtOAc/n-heptane gradient) followed by chiral preparative chromatography (Heptane:EtOH:DEA 35:65:0.2 gradient, chiracel AD-H) to give the first eluting enantiomer, **cbz-38a** (RT 2.61 min), and the second eluting enantiomer, **cbz-38b** (RT 2.99 min), both as a colourless oil. MW 473.62; $C_{30}H_{35}NO_4$. LCMS (6 minute method): m/z 456.3 $[M-H_2O+H]^+$ and 496.2 $[M+Na]^+$; $R_T$ 5.34min.

***1-[1,1'-Biphenyl]-2-yl-4-methyl-2-morpholin-2-ylpentan-2-ol hydrochloride (38a)***

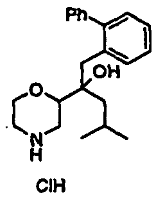

Palladium on carbon (10% weight) (0.4 g) is added to a stirring solution of **cbz-38a** (0.39 g, 0.84 mmol) with ammonium formate (0.53 g, 8.4 mmol) in ethanol (10 ml) at room temperature under nitrogen. The heterogeneous mixture is heated to reflux for 30 minutes, allowed to cool to room temperature and then filtered through a Celite pad. The filtrate is concentrated *in vacuo,* purified by ion exchange chromatography and then converted to the hydrochloride salt following *General Procedure 4* to give **38a** (0.25 g, 79%) as a yellow solid. MW 375.94; $C_{22}H_{29}NO_2$.HCl. $^1H$ NMR ($CD_3OD$) $\delta_H$ 7.48 (1H, bs), , 7.11-7.33 (8H, m), 3.87(1H, bs), 3.37-3.57 (2H, m), 3.25 (1H, s), 2.77-3.10 (5H, m), 1.54 (1H, s, br), 1.07-1.19 (1H, m), 0.93-1.00 (1H, m), 0.72 (3H, d, 6 Hz), 0.69 (3H, d, 6 Hz). LCMS (12 minute method): m/z 340.2 $[M+H]^+$, $R_T$ 5.30min.

**<u>Example 15H: Preparation of 1-(4-fluoro[1,1'-biphenyl]-2-yl)-4-methyl-2-morpholin-2-ylpentan-2-ol hydrochloride (40)</u>**

***1-(4-Fluoro[1,1'-biphenyl]-2-yl-4-methyl-2-[4-(phenylmethyl)morpholin-2-yl]pentan-2-ol (39)***

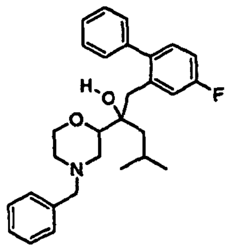

Compound **39** is prepared from **6** (0.95 g, 3.65 mmol) and 2-phenyl-5-fluoro benzyl magnesium bromide (0.5 M solution in diethyl ether, 1.2 eq) following *General Procedure 2*. 2-Phenyl-5-fluorobenzyl magnesium bromide is obtained from 2-phenyl-5-fluorobenzyl bromide following *General Procedure 5.* Excess 2-phenyl-5-fluoro benzyl magnesium bromide is subsequently added at room temperature and the reaction left stirring for 1 hour. Purification by flash column chromatography (EtOAc/cyclohexane 50 to 50%, gradient) gives **39** as a viscous oil (1.31 g, 80%): MW 447.60; $C_{29}H_{34}FNO_2$ LCMS: (6 minute method) m/z 448 $[M+H]^+$, $R_T$ 3.88 min.

***1-(4-Fluoro[1,1'-biphenyl]-2-yl)-4-methyl-2-morpholin-2-ylpentan-2-ol hydrochloride (40)***

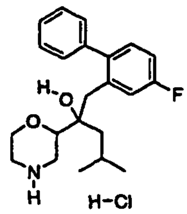

**40** is prepared from **39** (1.31 g, 2.92 mmol), α-chloroethyl chloroformate (0.9 ml) and solid supported Hünig's base (1.64 g) in anhydrous DCM (30 ml) following *General Procedure 3.* Purification by ion exchange ion exchange chromatography gives the free base of **40** as a viscous oil (0.71 g, 62%). After further purification using UV-guided preparative LCMS, the hydrochloride salt **40** (0.451 g, 39 %) is obtained following *General Procedure 4.* MW 393.95; $C_{22}H_{28}FNO_2$. HCl; $^1H$ NMR (DMSO-$d_6$): 9.16 (1H, s), 8.98 (1H, s), 7.44-7.32 (4H, m), 7.23-7.06 (4H, m), 3.83 (1H, dd, 12 Hz, 3 Hz), 3.59-3.50 (3H, m), 3.18 (1H, d, 12.5 Hz), 3.08 (1H, d, 12.5 Hz), 2.92-2.67 (4H, m), 1.54-1.40 (1H, m), 1.03 (1H, dd, 14.5 Hz, 5 Hz), 0.88 (1H, dd, 14.5 Hz, 6.5 Hz), 0.74 (3H, d, 6.5 Hz), 0.67 (3H, d, 6.5 Hz); LCMS: (12 min method) m/z 358 $[M-HCl+H]^+$ $R_T$5.47 min.

**Example 16H: Preparation of 1-cyclonentyl-2-[5-fluoro-2-(methyloxy)phenyl]-1-mornholin-2 ylethanol hydrochloride (42)**

***1-Cyclopentyl-2-[5-fluoro-2-(methyloxy)phenyl]-1-[4-(phenylmethyl)morpholin-2-yl]ethanol (41)***

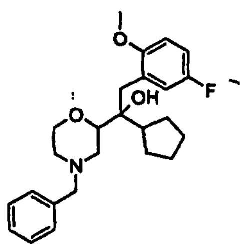

Compound **41** is obtained from 7 (0.7 g, 2.56 mmol) and 2-methoxy-5-fluorobenzyl magnesium bromide (5.63 ml, 2.82 mmol 1.1eq) in anhydrous THF (15 ml) following *General Procedure 2*. Further equivalents of 2-methoxy-5-fluorobenzyl magnesium bromide (8.49 ml, 4.25 mmol) are added. Purification by ion exchange chromatography gives **41** as a yellow oil (843 mg, 62% purity). MW 413.54; $C_{25}H_{32}FNO_3$. LCMS (6 minute method): m/z 414.2 $[M+H]^+$@ $R_T$ 4.11 min.

***1-Cyclopentyl-2-[5-fluoro-2-(methyloxy)phenyl]-1-morpholin-2-ylethanol hydrochloride (42)***

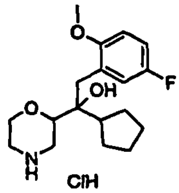

The free base of **42** is obtained from **41** (0.84 g, 2.04 mmol), α-chloroethyl chloroformate (0.88 ml, 8.16 mmol, 4eq) and polymer-supported Hünig's base (1.15 g, 4.08 mmol, 2eq) in DCM (15 ml) following *General Procedure 3*. Purification by ion exchange chromatography, followed by automated chromatography (5-20% DCM/MeOH gradient) and preparative LCMS and conversion into the hydrochloride salt following *General Procedure 4* gives **42** as a colourless gum (0.18 g, 14.1%). MW 359.82; $C_{18}H_{26}FNO_3$.HCl. $^1$H NMR ($CD_3OD$) $\delta_H$ 7.11-7.14 (1H, m), 6.95-6.97 (2H, m), 4.07-4.15 (1H, m), 3.67-3.75 (2H, m), 3.43 (1H, d, 12 Hz), 3.23 (3H, s), 3.22 (1H, d, 12 Hz), 2.92-3.10 (4H, m), 2.13-2.19 (1H, m), 1.42-1.73 (8H, m). LCMS (12 minute method): m/z 324.1 $[M-HCl+H]^+$, RT 4.83min.

**Example 17H: Preparation of 1-cyclopentyl-2-[2-(ethyloxy)phenyl]-1-morpholin-2-ylethanol hydrochloride (44)**

***1-Cyclopentyl-2-[2-(ethyloxy)phenyl]-1-[4-(phenylmethyl)morpholin-2-yl]-ethanol (43)***

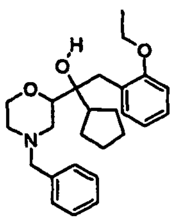

Compound **43** is obtained from **7** (2.09 g, 7.68 mmol) and 2-ethyloxy benzyl magnesium bromide (available from Reike Metals) (0.25 M solution in diethyl ether, 1.1 eq) following *General Procedure 2*. Purification by preparative LCMS followed by preparative LCMS gives **43** as viscous oil (0.691 g, 22 %). MW 409.57; $C_{26}H_{35}NO_3$; LCMS: (6 min method) m/z 410 $[M+H]^+$, Rt 3.8min.

***1-Cyclopentyl-2-[2-(ethyloxy)phenyl]-1-morpholin-2-ylethanol hydrochloride (44)***

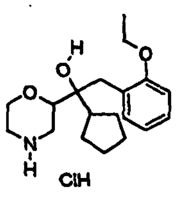

The free base of **44** is obtained from **43** (0.691 g, 1.69 mmol), α-chloroethyl chloroformate (0.80 ml) and solid

supported Hünig's base (0.95 g) in anhydrous DCM following *General Procedure 3*. Purification by ion exchange and conversion into its hydrochloride salt following *General Procedure 4* gives **44** (0.39 g, 65%) MW 355.91; $C_{19}H_{29}NO_3$.HCl; $^1H$ NMR ($CD_3OD$): 7.12-7.22 (2H, m), 6.82-6.88 (2H, m), 4.09-4.16 (3H, m), 3.69-3.80 (2H, m), 2.80-3.29 (6H, m), 2.04-2.10 (1H, m), 1.53-1.73 (11H, m); LCMS: (12 min method) m/z 320 $[M-HCl+H]^+$, Rt 5.03 min.

**Example 18H: Preparation of 1-Cyclopentyl-1-morpholin-2-yl-2-{2-(trifluoro methyl)oxy]phenyl}ethanol hydrochloride (46)**

***1-Cyclopentyl-2-[5-fluoro-2-(methyloxy)phenyl]-1-[4-(phenylmethyl) morpholin-2-yl]ethanol (45)***

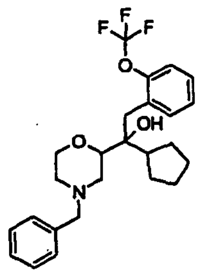

Compound **45** is obtained from **7** (0.6 g, 2.19 mmol) and commercially available (Fluorochem) 2-trifluoromethoxy-benzyl magnesium bromide (0.5M sol in diethylether, 4.8 ml, 2.41, mmol, 1.1 eq) in anhydrous THF (15 ml) following *General Procedure 2.* After addition of another 2 equivalents of 2-trifluoromethoxy-benzyl magnesium bromide and stirring for 2 hours at 0°C purification by ion exchange chromatography gives **45** (0.89g, 90%). MW 449.52; $C_{25}H_{30}F_3NO_3$. LCMS (6 minute method): m/z 450.2 $[M+H]^+$, $R_T$ 4.084 min.

***1-Cyclopentyl-1-morpholin-2-yl-2-{2-[(trifluoromethyl)oxy]phenyl} ethanol hydrochloride (46)***

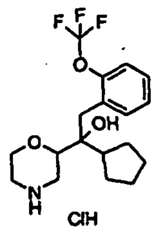

The free base of **46** is obtained from **45** (886 mg, 1.97 mmol), α-chloroethyl chloroformate (0.85 ml, 7.9 mmol, 4eq) and polymer-supported Hünig's base (1.11 g, 3.94 mmol, 2eq) in DCM (15 ml) following *General Procedure 3.* Purification by ion exchange chromatography followed by preparative LCMS (gradient) and conversion to its hydrochloride salt following *General Procedure 4* gives **46** as a gum (140 mg, 20%). MW 359.36; $C_{18}H_{24}F_3NO_3$.HCl. $^1H$ NMR ($CD_3OD$) $\delta_H$ 7.48-7.50 (1H, m), 7.14-7.25 (3H, m), 3.97 (1H, dd, 2.3 Hz, 12.5 Hz), 3.60-3.68 (2H, m), 3.27-3.31 (1H, m), 3.03 (2H, AB, 12.5 Hz), 2.73-2.97 (3H, m), 2.00-2.11 (1H, m), 1.30-1.63 (8H, m). LCMS (12 minute method): m/z 360.14 $[M-HCl+H]^+$, $R_T$ 5.14 min.

**Example 19H: Preparation of 2-[1,1'-biphenyl]-2-yl-1-cyclopentyl-1-morpholin-2-ylethanol hydrochloride (48)**

***2-[1,1'-Biphenyl]-2-yl-1-cyclopentyl-1-[4-(phenylmetltyl)morpholin-2-yl]ethanol (47a,47b)***

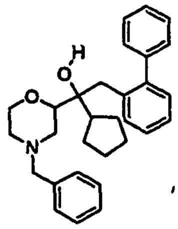

Compound **47** is prepared from **7** (1.27 g, 4.65 mmol) and 2-phenylbenzyl magnesium bromide (0.25 M solution in diethyl ether, 1.1 eq) following *General Procedure 2.* 2-Phenylbenzyl magnesium bromide is prepared from commercially available (Aldrich) 2-phenylbenzyl bromide following *General Procedure 5.* Purification by flash column chromatography (eluent: cyclohexane/ethyl acetate/ 90/10 [v/v]) gives **47a,47b** as viscous oil (1.75 g). **47a,47b** is taken onto the next step without further purification. MW 441.62; $C_{30}H_{35}NO_2$. LCMS: (6 min method) m/z 442 $[M+H]^+$, $R_T$3.51 min.

***2-[1,1'-Biphenyl]-2-yl-1-cyclopentyl-1-morpholin-2-ylethanol hydrochloride (48a,48b)***

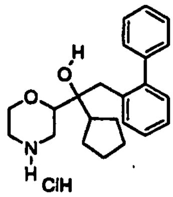

The free base of **48a, 48b** is prepared from **47a, 47b** (1.75 g, 3.95 mmol), solid supported Hünig's base (2.22 g) and α-chloroethyl chloroformate (1.62 ml) in anhydrous DCM (30 ml) following *General Procedure* 3. Purification by ion exchange chromatography followed by flash column chromatography (eluent: methanol/DCM 1/99 to 20/80 gradient) gives the free base as a viscous oil (805 mg, 58 %) which is converted into **48a, 48b** following *General Procedure 4.* MW 387.95; $C_{23}H_{29}NO_2$.HCl; $^1H$ NMR ($CD_3OD$): 7.66-7.40 (1H, m) 7.19-7.47 (8H, m), 3.92 (1H, dd, 13 Hz, 3.5 Hz), 3.59-3.67 (2H, m), 3.05-3.16 (4H, m), 2.93 (1H, td, 13 Hz, 3.5 Hz,), 2.59 (1H, t, 12 Hz), 1.98-1.88 (1H, m), 1.55-1.19 (8H, m). LCMS: (12 min method) m/z 351 $[M-HCl+H]^+$, $R_T$ 5.68 min.

**Example 20H: Preparation of 1-cyclopentyl-2-(4-fluoro[1,1'-biphenyl]-2-yl)-1-morpholin-2-ylethanol hydrochloride (50)**

***1-Cyclopentyl-2-(4-fluoro[1,1'-biphenyl]-2-yl)-1-[4-(phenylmethyl)morpholin-2-ylethanol*** *hydrochloride (49)*

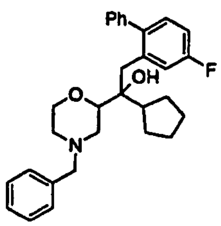

Compound **49** is obtained from **7** (0.9 g, 3.29 mmol) and 2-phenyl-5-fluorobenzyl magnesium bromide (0.5M solution in THF, 7.24 ml, 3.62 mmol) in anhydrous THF (20 ml) following *General Procedure 2.* 2-Phenyl-5-fluorobenzyl magnesium bromide is prepared from 2-phenyl-5-fluorobenzyl bromide following *General Procedure 5.* Further 2-phenyl-5-fluorobenzyl magnesium bromide is added after 30 min (0.3eq, 2 ml, 0.99 mmol). Purification by ion exchange chromatography followed by automated column chromatography (0-20% EtOAc/n-heptane gradient, 40 g) gives **49** (1.26 g, 83%) as a colourless liquid. MW 459.61; $C_{30}H_{34}FNO_2$. LCMS (6 minute method): m/z 460.5 $[M+H]^+$, $R_T$3.98min.

*1-Cyclopentyl-2-(4-fluoro[1,1'-biphenyl]-2-yl)-1-morpholin-2-ylethanol hydrochloride (50)*

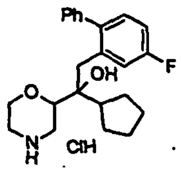

The free base of **50** is obtained from **49** (1.26 mg, 2.73 mmol), α-chloroethyl chloroformate (1.18 ml, 10.9 mmol) and polymer-supported Hünig's base (1.54 g, 5.47 mmol) in DCM (25 ml) following *General Procedure* 3. Purification by ion exchange chromatography followed by automated column chromatography (0-20% MeOH/ DCM gradient) and conversion to its hydrochloride salt gives **50** as a yellow solid (0.23 g, 23%). MW 405.94; $C_{23}H_{28}FNO_2$.HCl. $^1H$ NMR ($CD_3OD$) $\delta_H$ 7.20-7.37 (6H, m), 7.08-7.13 (1H, m), 6.91 (1H, td, 3.5 Hz, 8.5 Hz), 3.80 (1H, dd, 3.5 Hz, 13.0 Hz), 3.42-3.54 (2H, m), 2.99-3.06 (2H, m), 2.93 (2H, s), 2.83 (1H, td, 4.0 Hz, 12.5 Hz), 2.53 (1H, t, 12.0 Hz), 1.73-1.85 (1H, m), 1.12-1.44 (8H, m). LCMS (12 minute method): m/z 370.2 $[M-HCl+H]^+$, $R_T$ 5.46min.

<u>**Example 21H: Preparation of 2-[5-fluoro-2-methyloxy)phenyl]-1-morpholin-2-yl-1-tetrahydro-2H-pyran-4-ylethanol hydrochloride (52)**</u>

*2-[5-Fluoro-2-(methyloxy)phenyl]-1-[4-(phenylmethyl)morpholin-2-yl]-1-tetrahydro-2H-pyran-4-ylethanol (51)*

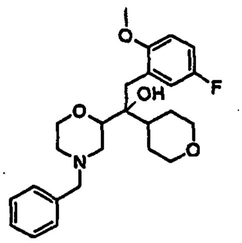

Compound **51** is obtained from **8** (0.6 g, 2.07 mmol) and 2-methoxy-5-fluorobenzyl magnesium bromide (4.6 ml, 2.28 mmol, 1.1eq) in anhydrous THF (15 ml) following *General Procedure* 2. Further equivalents of 2-methoxy-5-fluorobenzyl magnesium bromide (8.28 ml, 4.14 mmol) are added and the mixture is warmed to room temperature and left stirring over night. Purification by ion exchange chromatography followed by automated chromatography (10-70% n-heptane/EtOAc gradient) gives **51** as a colourless oil (375 mg, 42%). MW 429.54, $C_{25}H_{32}FNO_4$. LCMS (6 minute method): m/z 430.2 $[M+H]^+$, $R_T$ 3.12 min.

*2-[5-Fluoro-2-methyloxy)phenyl]-1-morpholin-2-yl-1-tetrahydro-2H-pyran-4-ylethanol hydrochloride (52)*

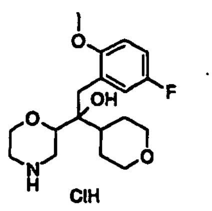

The free base of **52** is obtained from **51** (0.31 g, 0.73 mmol), α-chloroethyl chloroformate (0.31 ml, 2.9 mmol) and polymer-supported Hünig's base (0.41 g, 1.45 mmol) in DCM (7 ml) following *General Procedure 3.* Purification by ion exchange chromatography and conversion to the hydrochloride salt following *General Procedure 4* gives **52** as a white solid (0.19 g, 77%). MW 375.82; $C_{18}H_{26}FNO_4$.HCl. $^1$H NMR ($CD_3OD$) $\delta_H$ 6.98-7.01 (1H, m), 6.83-6.86 (2H, m), 3.99 (1H,dd, 3.5 Hz, 13 Hz), 3.82- 3.87 (2H, m), 3.63-3.73 (5H, m), 3.12-3.33 (4H, m), 2.91-3.02 (2H, m), 2.81 (2H, AB, 14 Hz), 1.31-1.73 (5H, m). LCMS (12 minute method): m/z 340.2 $[M-HCl+H]^+$, $R_T$ 3.78 min.

**Example 22H: Preparation of 1-morpholin-2-yl-1-tetrahydro-2H-oyran-4-yl-2-{2-[(trifluoromethyl)oxy]phenyl} ethanol hydrochloride (54)**

***1-[4-(Phenylmethyl)morpholin-2-yl]-l-1-tetrahydro-2H-pyran-4-yl-2-{2-[(trifluoromethyl)oxy]phenyl}ethanol (53)***

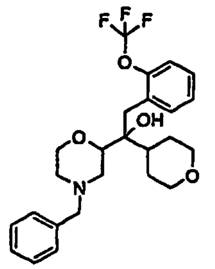

Compound **53** is obtained from **8** (0.61 g, 2.11 mmol) and commercially available (Fluorochem) 2-trifluoromethoxy benzyl magnesium bromide (4.6 ml, 2.32 mmol, 1.1eq) in anhydrous THF (15 ml) following *General Procedure 2*. A further half equivalent of 2-trifluoromethoxy benzyl magnesium bromide (4.22 ml, 2.11 mmol) is added. Purification by ion exchange chromatography gives **53** as an oil of 88% purity (1.39 g isolated material) which is directly used in the next step. MW 465.52; $C_{25}H_{30}F_3NO_4$. LCMS (6 minute method): m/z 466.2 $[M+H]^+$, $R_T$ 3.67 min.

***1-Morpholin-2-yl-1-tetrahydro-2H-pyran-4-yl-2-{2-[(trifluoromethyl)oxy] phenyl}ethanol hydrochloride (54)***

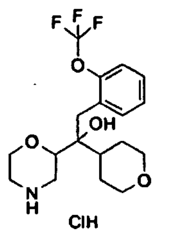

The free base of **54** is obtained from **53** (0.27 g, 0.57 mmol), α-chloroethyl chloroformate (0.25 ml, 2.30 mmol, 4eq) and polymer-supported Hünig's base (0.32 g, 1.15 mmol, 2eq) in DCM (5 ml) following *General Procedure 3.* Purification by ion exchange chromatography followed by preparative LCMS (gradient) and conversion to the hydrochloride salt following *General Procedure 4* gives **54** as white solid (82 mg, 17%). MW 411.85; $C_{18}H_{24}F_3NO_4$.HCl. $^1$H NMR ($CD_3OD$) $\delta_H$ 7.45-7.48 (1H, m), 7.16-7.27 (3H, m), 3.98 (1H, dd, 4.0 Hz, 13 Hz), 3.65-3.88 (4H, m), 3.12-3.31 (4H, m), 2.87-3.01 (4H, m), 1.30-1.68 (5H, m). LCMS (12 minute method): m/z 376.1 $[M+H]^+$, $R_T$ 4.28 min.

The pharmacological profile of the compounds of Formula (IH) can be demonstrated as follows. The preferred exemplified compounds above exhibit a $K_i$ value less than 1 μm, more preferably less than 500nM at the norepinephrine transporter as determined using the scintillation proximity assay described below. Furthermore, the preferred exemplified compounds above selectively inhibit the norepinephrine transporter relative to the serotonin and dopamine transporters by a factor of at least five using the scintillation proximity assays as described below.

**Generation of stable cell-lines expressing the human dopamine, norepinephrine and serotonin transporters**

Standard molecular cloning techniques are used to generate stable cell-lines expressing the human dopamine, norepinephrine, and serotonin transporters. The polymerase chain reaction (PCR) was used in order to isolate and amplify each of the three full-length cDNAs from an appropriate cDNA library. Primers for PCR were designed using the following published sequence data:

Human dopamine transporter: GenBank M95167. Reference: Vandenbergh DJ, Persico AM and Uh1 GR. A human dopamine transporter cDNA predicts reduced glycosylation, displays a novel repetitive element and provides racially-dimorphic TaqI RFLPs. Molecular Brain Research (1992) Volume 15, pages 161-166.
Human norepinephrine transporter: GenBank M65105. Reference: Pacholczyk T, Blakely, RD and Amara SG. Expression cloning of a cocaine- and antidepressant-sensitive human noradrenaline transporter. Nature (1991) Volume 350, pages 350-354.
Human serotonin transporter: GenBank L05568. Reference: Ramamoorthy S, Bauman AL, Moore KR, Han H, Yang-Feng T, Chang AS, Ganapathy V and Blakely RD. Antidepressant- and cocaine-sensitivehuman serotonin transporter: Molecular cloning, expression, and chromosomal localization Proceedings of the National Academy of Sciences of the USA (1993) Volume 90, pages 2542-2546.
The PCR products are cloned into a mammalian expression vector (e.g., pcDNA3.1 (Invitrogen)) using standard ligation techniques. The constructs are then used to stably transfect HEK293 cells using a commercially available lipofection reagent (Lipofectamine™ - Invitrogen) following the manufacturer's protocol.

**Scintillation proximity assays for determining the affinity of test ligands at the norepinephrine transporter**

The compounds of use in the present invention are norepinephrine reuptake inhibitors, and possess excellent activity in, for example, a scintillation proximity assay (e.g., J. Gobel, D.L. Saussy and A. Goetz, J. Pharmacol. Toxicol. (1999) 42:237-244). Thus, $^3$H-nisoxetine binding to norepinephrine re-uptake sites in a cell line transfected with DNA encoding human norepinephrine transporter binding protein has been used to determine the affinity of ligands at the norepinephrine transporter.

**Membrane Preparation:**

Cell pastes from large scale production of HEK-293 cells expressing cloned human norepinephrine transporters were homogenized in 4 volumes 50mM Tris-HCl containing 300mM NaCl and 5mM KCl, pH 7.4. The homogenate was centrifuged twice (40,000g, 10min, 4°C) with pellet re-suspension in 4 volumes of Tris-HCl buffer containing the above reagents after the first spin and 8 volumes after the second spin. The suspended homogenate was centrifuged (100g, 10min, 4°C) and the supernatant kept and re-centrifuged (40,000g, 20min, 4°C). The pellet was resuspended in Tris-HCl buffer containing the above reagents along with 10%w/v sucrose and 0.1mM phenylmethylsulfonyl fluoride (PMSF). The membrane preparation was stored in aliquots (1ml) at -80°C until required. The protein concentration of the membrane preparation was determined using a bicinchoninic acid (BCA) protein assay reagent kit (available from Pierce).

**[$^3$H]-Nisoxetine Binding Assay:**

Each well of a 96 well microtitre plate was set up to contain the following:

| | |
|---|---|
| 50μl | 2nM [N-methyl-$^3$H]-Nisoxetine hydrochloride (70-87Ci/mmol, from NEN Life Science Products) |
| 75μl | Assay buffer (50mM Tris-HCl pH 7.4 containing 300mM NaCl and 5mM KCl) |
| 25μl | Test compound, assay buffer (total binding) or 10μM Desipramine HCl (non-specific binding) |
| 50μl | Wheatgerm agglutinin coated poly (vinyltoluene) (WGA PVT) SPA Beads (Amersham Biosciences RPNQ0001) (10mg/ml) |
| 50μl | Membrane (0.2mg protein per ml) |

The microtitre plates were incubated at room temperature for 10 hours prior to reading in a Trilux scintillation counter. The results were analysed using an automatic spline fitting programme (Multicalc, Packard, Milton Keynes, UK) to provide Ki values for each of the test compounds.

## Serotonin Binding Assay

The ability of a test compound to compete with [$^3$H]-citalopram for its binding sites on cloned human serotonin transporter containing membranes has been used as a measure of test compound ability to block serotonin uptake via its specific transporter (Ramamoorthy, S., Giovanetti, E., Qian, Y., Blakely, R., (1998) J. Biol. Chem. 273: 2458).

**Membrane Preparation:**

Membrane preparation is essentially similar to that for the norepinephrine transporter containing membranes as described above. The membrane preparation was stored in aliquots (1ml) at -70°C until required. The protein concentration of the membrane preparation was determined using a BCA protein assay reagent kit.

**[$^3$H]-Citalopram Binding Assay:**

Each well of a 96 well microtitre plate was set up to contain the following:

| | |
|---|---|
| 50μl | 2nM [$^3$H]-Citalopram (60-86Ci/mmol, Amersham Biosciences) |
| 75μl | Assay buffer (50mM Tris-HCl pH 7.4 containing 150mM NaCl and 5mM KCl) |
| 25μl | Diluted compound, assay buffer (total binding) or 100μM Fluoxetine (non-specific binding) |
| 50μl | WGA PVT SPA Beads (40mg/ml) |
| 50μl | Membrane preparation (0.4mg protein per ml) |

The microtitre plates were incubated at room temperature for 10 hours prior to reading in a Trilux scintillation counter. The results were analysed using an automatic spline fitting programme (Multicalc, Packard, Milton Keynes, UK) to provide Ki (nM) values for each of the test compounds.

## Dopamine Binding Assay

The ability of a test compound to compete with [$^3$H]-WIN35,428 for its binding sites on human cell membranes containing cloned human dopamine transporter has been used as a measure of the ability of such test compounds to block dopamine uptake via its specific transporter (Ramamoorthy et al 1998 *supra*).

**Membrane Preparation:**

Is essentially the same as for membranes containing cloned human serotonin transporter as described above.

**[$^3$H]-WIN35,428 Binding Assay:**

Each well of a 96well microtitre plate was set up to contain the following:

| | |
|---|---|
| 50μl | 4nM [$^3$H]-WIN35,428 (84-87Ci/mmol, from NEN Life Science Products) |
| 75μl | Assay buffer (50mM Tris-HCl pH 7.4 containing 150mM NaCl and 5mM KCl) |
| 25μl | Diluted compound, assay buffer (total binding) or 100μM Nomifensine (non-specific binding) |
| 50μl | WGA PVT SPA Beads (10mg/ml) |
| 50μl | Membrane preparation (0.2mg protein per ml.) |

The microtitre plates were incubated at room temperature for 120 minutes prior to reading in a Trilux scintillation counter. The results were analysed using an automatic spline fitting programme (Multicalc, Packard, Milton Keynes; UK) to provide Ki values for each of the test compounds.

## Acid Stability

The acid stability of a compound according to the present invention was determined as a solution in buffer at 6 different pH values (HCl 0.1N, pH 2, pH 4, pH 6, pH 7, and pH 8) at 40°C over a time course of 72 hours. Samples were taken at the beginning of the study and after 3,6 and 24 hours and analysed by capillary electrophoresis. The original sample used in this study contained 0.8% of the undesired epimer as internal standard. The samples taken at

the different time points during the study did not show any significant change in the percentage of the undesired epimer. This assay confirms that compounds of the present invention are chemically and configurationally stable under acidic conditions.

**In Vitro Determination of the Interaction of compounds with CYP2D6 in Human Hepatic Microsomes**

Cytochrome P450 2D6 (CYP2D6) is a mammalian enzyme which is commonly associated with the metabolism of around 30% of pharmaceutical compounds. Moreover, this enzyme exhibits genetic polymorphism, resulting in the presence of both normal and poor metabolizers in the population. A low involvement of CYP2D6 in the metabolism of compounds (i.e. the compound being a poor substrate of CYP2D6) is desirable in order to reduce any variability from subject to subject in the pharmacokinetics of the compound. Also, compounds with a low inhihibitor potential for CYP2D6 are desirable in order to avoid drug-drug interactions with co-administered drugs that are substrates of CYP2D6. Compounds can be tested both as substrates and as inhibitors of this enzyme by means of the following assays.

**CYP2D6 substrate assay**

**Principle:**

This assay determines the extent of the CYP2D6 enzyme involvement in the total oxidative metabolism of a compound in microsomes. Preferred compounds of the present invention exhibit less than 75% total metabolism via the CYP2D6 pathway.

For this in vitro assay, the extent of oxidative metabolism in human liver microsomes (HLM) is determined after a 30 minute incubation in the absence and presence of Quinidine, a specific chemical inhibitor of CYP2D6. The difference in the extent of metabolism in absence and presence of the inhibitor indicates the involvement of CYP2D6 in the metabolism of the compound.

**Materials and Methods:**

Human liver microsomes (mixture of 20 different donors, mixed gender) were acquired from Human Biologics (Scottsdale, AZ, USA). Quinidine and β-NADPH β-Nicotinamide Adenine Dinucleotide Phosphate, reduced form, tetrasodium salt) were purchased from Sigma (St Louis, MO, USA). All the other reagents and solvents were of analytical grade. A stock solution of the new chemical entity (NCE) was prepared in a mixture of Acetonitrile/Water to reach a final concentration of acetonitrile in the incubation below 0.5%.

The microsomal incubation mixture (total volume 0.1 mL) contained the NCE (4 μM), β-NADPH (1 mM), microsomal proteins (0.5 mg/mL), and Quinidine (0 or 2 μM) in 100 mM sodium phosphate buffer pH 7.4. The mixture was incubated for 30 minutes at 37 °C in a shaking waterbath. The reaction was terminated by the addition of acetonitrile (75 μL). The samples were vortexed and the denaturated proteins were removed by centrifugation. The amount of NCE in the supernatant was analyzed by liquid chromatography /mass spectrometry (LC/MS) after addition of an internal standard. A sample was also taken at the start of the incubation (t=0), and analysed similarly.

Analysis of the NCE was performed by liquid chromatography /mass spectrometry. Ten μL of diluted samples (20 fold dilution in the mobile phase) were injected onto a Spherisorb CN Column, 5 μM and 2.1 mm x 100 mm (Waters corp. Milford, MA, USA). The mobile phase consisting of a mixture of Solvent A/Solvent B, 30/70 (v/v) was pumped (Alliance 2795, Waters corp. Milford, MA, USA) through the column at a flow rate of 0.2 ml/minute. Solvent A and Solvent B were a mixture of ammonium formate $5.10^{-3}$ M pH 4.5/ methanol in the proportions 95/5 (v/v) and 10/90 (v/v), for solvent A and solvent B, respectively. The NCE and the internal standard were quantified by monitoring their molecular ion using a mass spectrometer ZMD or ZQ (Waters-Micromass corp, Machester, UK) operated in a positive electrospray ionisation.

The extent of CYP2D6 involvement (% of CYP2D6 involvement) was calculated comparing the extent of metabolism in absence and in presence of quinidine in the incubation.

The extent of metabolism without inhibitor (%) was calculated as follows:

$$\frac{(\text{NCE response in samples without inhibitor})_{\text{time 0}} - (\text{NCE response in samples without inhibitor})_{\text{time 30}}}{(\text{NCE response in samples without inhibitor})_{\text{time 0}}} \times 100$$

The extent of metabolism with inhibitor (%) was calculated as follows:

$$\frac{(\text{NCE response in samples without inhibitor})_{\text{time 0}} - (\text{NCE response in samples with inhibitor})_{\text{time 30}}}{(\text{NCE response in samples without inhibitor})_{\text{time 0}}} \times 100$$

where the NCE response is the area of the NCE divided by the area of the internal standard in the LC/MS analysis chromatogram, time0 and time30 correspond to the 0 and 30 minutes incubation time.

The % of CYP2D6 involvement was calculated as follows :

$$\frac{(\%\ \text{extent of metabolism without inhibitor}) - (\%\ \text{extent of metabolism with inhibitor})}{\%\ \text{extent of metabolism without inhibitor}} \times 100$$

**CYP2D6 inhibitor assay**

**Principle:**

The CYP2D6 inhibitor assay evaluates the potential for a compound to inhibit CYP2D6. This is performed by the measurement of the inhibition of the bufuralol 1'-hydroxylase activity by the compound compared to a control. The 1'-hydroxylation of bufuralol is a metabolic reaction specific to CYP2D6. Preferred compounds of the present invention exhibit an $IC_{50}$ higher than 6 μM for CYP2D6 activity, the $IC_{50}$ being the concentration of the compound that gives 50 % of inhibition of the CYP2D6 activity.

**Materials and Methods:**

Human liver microsomes (mixture of 20 different donors, mixed gender) were acquired from Human Biologics (Scottsdale, AZ). β-NADPH was purchased from Sigma (St Louis, MO). Bufuralol was purchased from Ultrafine (Manchester, UK). All the other reagents and solvents were of analytical grade.

Microsomal incubation mixture (total volume 0.1 mL) contained bufuralol 10 μM, β-NADPH (2 mM), microsomal proteins (0.5 mg/mL), and the new chemical entity (NCE) (0, 5, and 25 μM) in 100 mM sodium phosphate buffer pH 7.4. The mixture was incubated in a shaking waterbath at 37 °C for 5 minutes. The reaction was terminated by the addition of methanol (75 μL). The samples were vortexed and the denaturated proteins were removed by centrifugation. The supernatant was analyzed by liquid chromatography connected to a fluorescence detector. The formation of the 1'-hydroxybufuralol was monitored in control samples (0 μM NCE) and in the samples incubated in presence of the NCE. The stock solution of NCE was prepared in a mixture of Acetonitrile/Water to reach a final concentration of acetonitrile in the incubation below 1.0%.

The determination of l'hydroxybufuralol in the samples was performed by liquid chromatograhy with fluorimetric detection as described below. Twenty five μL samples were injected onto a Chromolith Performance RP-18e column (100 mm x 4.6 mm) (Merck KGAa, Darmstadt, Germany). The mobile phase, consisting of a mixture of solvent A and solvent B whose the proportions changed according the following linear gradient, was pumped through the column at a flow rate of 1 ml/min:

| Time (minutes) | Solvent A (%) | Solvent B (%) |
|---|---|---|
| 0 | 65 | 35 |
| 2.0 | 65 | 35 |
| 2.5 | 0 | 100 |
| 5.5 | 0 | 100 |
| 6.0 | 65 | 35 |

Solvent A and Solvent B consisted of a mixture of 0.02 M potassium dihydrogenophosphate buffer pH3/ methanol in the proportion 90/10 (v/v) for solvent A and 10/90 (v/v) for solvent B. The run time was 7.5 minutes. Formation of 1'-hydroxybufuralol was monitored by fluorimetric detection with extinction at λ 252 nm and emission at λ 302 nm.

The $IC_{50}$ of the NCE for CYP2D6 was calculated by the measurement of the percent of inhibition of the formation of the 1'-hydroxybufuralol in presence of the NCE compared to control samples (no NCE) at a known concentration of the NCE.

The percent of inhibition of the formation of the 1'-hydroxybufuralol is calculated as follows:

$$\frac{\text{(1'-hydroxybufuralol formed without inhibitor)} - \text{(1'-hydroxybufuralol formed with inhibitor)}}{\text{(1'-hydroxybufuralol area formed without inhibitor)}} \times 100$$

The $IC_{50}$ is calculated from the percent inhibition of the formation of the 1'-hydroxybufuralol as follows (assuming competitive inhibition):

$$\frac{\text{NCE Concentration} \times (100 - \text{Percent of inhibition})}{\text{Percent of inhibition}}$$

The $IC_{50}$ estimation is assumed valid if inhibition is between 20% and 80% (Moody GC, Griffin SJ, Mather AN, McGinnity DF, Riley RJ. (1999) Fully automated analysis of activities catalyzed by the major human liver cytochrome P450 (CYP) enzymes: assessment of human CYP inhibition potential. Xenobiotica 29(1): 53-75).

**Activity of Norepinephrine Reuptake Inhibitors in The Morphine Withdrawal, Rat Hot Flush Model**

Simpkins et al. ((1983) Life Sci. 32:1957-1966) first published morphine withdrawal in the rat as a putative model for hot flushes, based on observations highlighting the similarity of symptoms of gonadal steroid withdrawal to those of opioid withdrawal. Although less severe, the signs and symptoms associated with clinical hot flushes, or estrogen deficiency, in the rat parallel those produced by naloxone-precipitated withdrawal in morphine dependent rats, including: 1) an increase in tail skin temperature; 2) a surge in luteinizing hormone; and 3) an increase in heart rate. Each of these responses is associated with an increase in sympathetic outflow, which is a current mechanistic hypothesis for hot flushes. As a corollary, morphine addicted humans show a withdrawal pattern suggesting increased sympathetic outflow and symptoms that include hot flushes.

A key requirement of animal models is that they mimic the treatment efficacy observed with the human disease. The morphine withdrawal hot flush model, either in its originally described form, or with the modifications described herein, is responsive to agents typically used in the treatment of human hot flushes. This includes various forms of estrogen (Simpkins et al. (1983) *supra),* clonidine (data not shown), tibolone (data not shown), and medroxyprogesterone (data not shown). Furthermore, the model is sensitive to agents known to be associated with the induction of hot flushes in postmenopausal women, i.e., raloxifene (Merchenthaler et al. (1998) Maturitas 30: 307-316).

In this example, a modification of the original procedure of Simpkins et al. (1983) is used, employing ovariectomized Sprague-Dawley rats. Animals at 60 days of age (or 200-225 grams) are ovariectomized, and allowed a 14-day rest period to insure surgical recovery and clearance of endogenous ovarian hormones. Test compound administration (po or sc) is initiated on day 14 post-ovariectomy in a volume of 1 ml/kg. Once daily adminstration of test compounds continues through the end of the experiment. On days 15 and 17 post-ovariectomy, the rats are lightly anesthetized with isoflurane and a single 75 mg morphine (free base) pellet is surgically implanted subcutaneously.

On day 21 post-ovariectomy, animals are given ketamine (80mg/kg; IM) 2 hours after final administration of the test compound. Following induction of the anesthesia, rats are then placed in individual plexiglass cages and temperature sensitive probes are applied to the dorsal side of the tail base. Temperature monitoring is initiated 30 minutes after administration of ketamine, and is recorded every 15 seconds for a 1-hr period. To induce morphine withdrawal, 1mg/kg naloxone is given subcutaneously 15 minutes after start of temperature monitoring. A sharp rise in tail skin temperature typically occurs within 5 minutes post-naloxone injection, and two quantitative endpoints are made: 1) tail skin temperature at 15 minutes post-naloxone, and 2) area under the temperature response curve for the 45 minute post-naloxone measurement period.

Compounds are dosed either subcutaneously or orally at the doses listed in Table 1, below. Ethinyl estradiol is dosed orally at 0.3 mg/kg as a positive control. The selective norepinephrine reuptake inhibitors desipramine, (R, S)-reboxetine, and (*R*)-thionisoxetine which do not form part of the invention (Gehlert et al. (1995) Life Sci. 56(22): 1915-1920) are tested. Using area under the curve measurements, the response induced by each compound is calculated as a percent inhibition of temperature increase versus the morphine control. Ethinyl estradiol generally inhibits the temperature increase by 70-90%. At 10 mg/kg, desipramine shows a 101% inhibition, reboxetine (5 mg/kg) shows a 94% inhibition, and (*R*)-thionisoxetine (5 mg/kg) shows a 101% inhibition compared to the morphine control.

Table 1

| Compound | Route of Administration | Dose | % Inhibition vs. Morphine Control |
|---|---|---|---|
| Ethinyl Estradiol | PO | 0.3 mg/kg | 89, 73, 90 |
| Desipramine | PO | 10 mg/kg | 101% |
| (*R*,*S*)-Reboxetine | SC | 5 mg/kg | 94% |
| (*R*)-thionisoxetine | SC | 5 mg/kg | 101% |

**Double-Blind, Placebo-Controlled Study to Determine the Effect of Selective Norepinephrine Reuptake Inhibitors on Patients Suffering Hormonal Variation and**

**Hot Flashes**

Approximately twenty patients diagnosed as suffering from hormonal variation and hot flashes receive either a selective norepinephrine reuptake inhibitor or a placebo. Each subject participates in six randomized test periods. In three of the test periods, each subject is given a selective norepinephrine reuptake inhibitor, and in the other three test periods, is given a placebo. Efficacy of the test compound is assessed by reference to immunological profile, rating scales, checklists and diminishment of the attendant disease state.

The results of this study would demonstrate the effectiveness of a selective norepinephrine reuptake inhibitor with respect to placebo in the treatment or prevention of hot flashes following drug treatment.

## Claims

1. Use of a selective norepinephrine reuptake inhibitor for the manufacture of a medicament for the treatment of hot flashes or vasomotor symptoms, wherein said selective norepinephrine reuptake inhibitor is a compound of formula (IH):

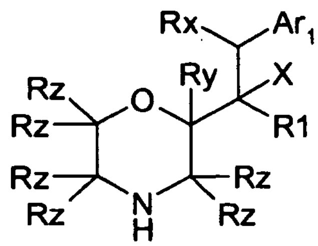

(IH)

wherein,

X is OH, C1-C4 alkoxy, $NH_2$ or NH(C1-C4 alkyl);
Rx is H or C1-C4 alkyl;
Ry is H or C1-C4 alkyl;
each Rz group is independently H or C1-C4 alkyl, with the proviso that not more than 3 Rz groups may be C1-C4 alkyl;
R1 is C1-C6 alkyl (optionally substituted with 1, 2 or 3 halogen atoms and/or with 1 substituent selected from C1-C4 alkylthio (optionally substituted with 1, 2 or 3 fluorine atoms), C1-C4 alkoxy (optionally substituted with 1, 2 or 3 fluorine atoms), C3-C6 cycloalkoxy, C1-C4 alkylsulfonyl, cyano, -CO-O(C1-C2 alkyl), -O-CO-(C1-C2 alkyl) and hydroxy); C2-C6 alkenyl (optionally substituted with 1, 2 or 3 halogen atoms); C3-C6 cycloalkyl (optionally substituted with 1, 2 or 3 halogen atoms and/or with 1 substituent selected from C 1-C4 alkoxy and hydroxy) wherein one C-C bond within the cycloalkyl moiety is optionally substituted by an O-C, S-C or C=C bond; C4-C7 cycloalkylalkyl (optionally substituted with l, 2 or 3 halogen atoms and/or with 1 substituent selected from C1-C4 alkoxy and hydroxy) wherein one C-C bond within the cycloalkyl moiety is optionally substituted by

an O-C, S-C or C=C bond; or $CH_2Ar2$; and
Ar1 and Ar2 are each independently a phenyl ring or a 5- or 6-membered heteroaryl ring each of which is optionally substituted with 1, 2 or 3 substituents (depending upon the number of available substitution positions) each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), -CO-O(C1-C4 alkyl), cyano, - NRR, -CONRR, halo and hydroxy and/or with 1 substituent selected from pyridyl, thiophenyl, phenyl, benzyl and phenoxy each of which is optionally ring-substituted with 1, 2 or 3 substituents each independently selected from halogen, C1-C4 alkyl (optionally substituted with 1, 2 or 3 halogen atoms), C1-C4 alkoxy (optionally substituted with 1, 2 or 3 halogen atoms), carboxy, nitro, hydroxy, cyano, -NRR, - CONRR, $SO_2NRR$ and $SO_2R$); and
each R is independently H or C1-C4 alkyl;
or a pharmaceutically acceptable salt thereof.

**2.** The use according to Claim 1, wherein said selective norepinephrine reuptake inhibitor is a compound of the formula (IIIH)

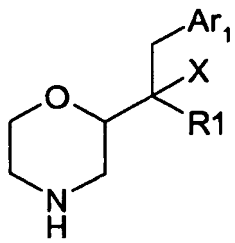

**(IIIH)**

wherein, X, R1 and Ar1 are as defined in Claim 1;
or a pharmaceutically acceptable salt thereof.

**3.** The use according to Claim 1, wherein said selective norepinephrine reuptake inhibitor is a compound of the formula (IVH)

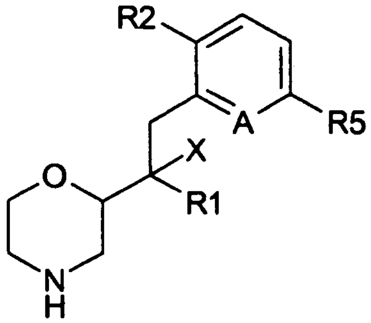

**(IVH)**

wherein,

X is OH or $NH_2$;
R1 is C1-C6 alkyl (optionally substituted with 1, 2 or 3 halogen atoms and/or with 1 substituent selected from C1-C4 alkoxy (optionally substituted with 1, 2 or 3 fluorine atoms), cyano, and hydroxy); C3-C6 cycloalkyl (optionally substituted with 1, 2 or 3 halogen atoms and/or with 1 substituent selected from C1-C4 alkoxy and hydroxy) wherein one C-C bond within the cycloalkyl moiety is optionally substituted by an O-C bond; or $CH_2Ar2$ wherein Ar2 is a phenyl ring optionally substituted with 1, 2 or 3 substituents each independently selected from C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo and hydroxy;

A is N or CH;
R2 is C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo, hydroxy, pyridyl, thiophenyl, phenyl (optionally substituted with 1, 2 or 3 substituents each independently selected from halogen, C1-C4 alkyl (optionally substituted with 1, 2 or 3 halogen atoms), or C1-C4 alkoxy (optionally substituted with 1, 2 or 3 halogen atoms)) or phenoxy (optionally substituted with 1, 2 or 3 halogen atoms);
R5 is H, C1-C4 alkyl (optionally substituted with 1, 2 or 3 halogen atoms), C1-C4 alkoxy (optionally substituted with 1, 2 or 3 halogen atoms), C1-C4 alkylthio (optionally substituted with 1, 2 or 3 halogen atoms), halo or hydroxy;

or a pharmaceutically acceptable salt thereof.

**4.** The use according to Claim 1, wherein said selective norepinephrine reuptake inhibitor is a compound of the formula (VH)

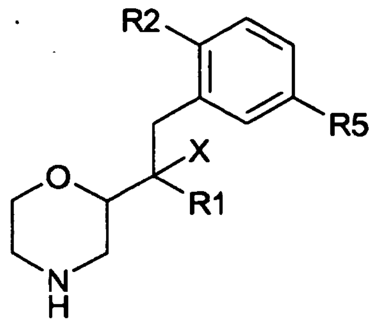

(VH)

wherein,

X is OH or $NH_2$;
R1 is C1-C6 alkyl (optionally substituted with 1, 2 or 3 fluorine atoms), C3-C6 cycloalkyl wherein one C-C bond within the cycloalkyl moiety is optionally substituted by an O-C bond or $CH_2Ar2$ wherein Ar2 is a phenyl ring optionally substituted with 1 or 2 substituents each independently selected from C1-C4 alkyl (optionally substituted with 1, 2 or 3 halogen atoms), C1-C4 alkoxy (optionally substituted with 1, 2 or 3 halogen atoms), halo and hydroxy;
R2 is C1-C4 alkyl (optionally substituted with 1, 2 or 3 fluorine atoms), C1-C4 alkoxy (optionally substituted with 1, 2 or 3 fluorine atoms) or phenyl (optionally substituted with 1, 2 or 3 fluorine atoms);
and R5 is H or F;

or a pharmaceutically acceptable salt thereof.

**5.** The use according to Claim 1, wherein said selective norepinephrine reuptake inhibitor is a compound of the formula (VIH)

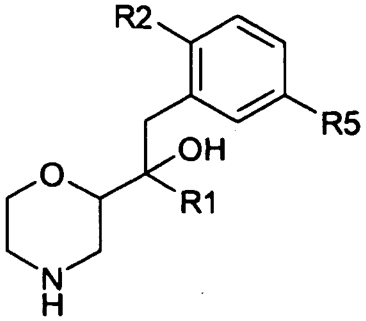

(VIH)

wherein,

R1 is C1-C6 alkyl (optionally substituted with 1, 2 or 3 fluorine atoms) or C3-C6 cycloalkyl wherein one C-C bond within the cycloalkyl moiety is optionally substituted by an O-C bond;
R2 is C1-C4 alkyl (optionally substituted with 1, 2 or 3 fluorine atoms), C1-C4 alkoxy (optionally substituted with 1, 2 or 3 fluorine atoms) or phenyl (optionally substituted with 1, 2 or 3 fluorine atoms);
and R5 is H or F;

or a pharmaceutically acceptable salt thereof.

6. The use according to Claim 1, wherein said selective norepinephrine reuptake inhibitor is a compound of the formula:

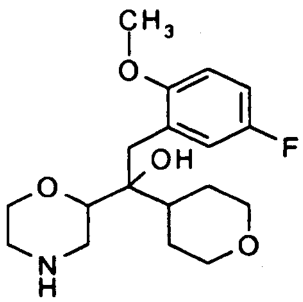

or the hydrochloride salt thereof.

**Patentansprüche**

1. Verwendung eines selektiven Wiederaufnahmeinhibitors von Norepinephrin zur Herstellung eines Arzneimittels für die Behandlung von Hitzewallungen oder vasomotorischen Symptomen, worin der selektive Wiederaufnahmeinhibitor von Norepinephrin eine Verbindung der Formel (IH) ist

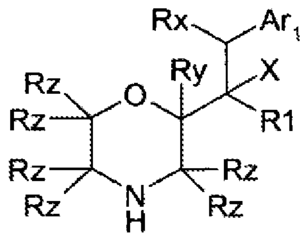

(IH)

worin

X für OH, $C_1$-$C_4$-Alkoxy, $NH_2$ oder NH-($C_1$-$C_4$-Alkyl) steht,
Rx für H oder $C_1$-$C_4$-Alkyl steht,
Ry für H oder $C_1$-$C_4$-Alkyl steht,
Rz jeweils unabhängig für H oder $C_1$-$C_4$-Alkyl steht, mit der Maßgabe, dass nicht mehr als 3 der Gruppen Rz $C_1$-$C_4$-Alkyl sein können,
R1 steht für $C_1$-$C_6$-Alkyl (das optional substituiert ist mit 1, 2 oder 3 Halogenatomen und/oder mit 1 Substituenten, der aus $C_1$-$C_4$-Alkylthio ausgewählt ist (das optional substituiert ist mit 1, 2 oder 3 Fluoratomen), $C_1$-$C_4$-Alkoxy (das optional substituiert ist mit 1, 2 oder 3 Fluoratomen), $C_3$-$C_6$-Cycloalkoxy, $C_1$-$C_4$-Alkylsulfonyl, Cyano, -CO-O-($C_1$-$C_2$-Alkyl), -O-CO-($C_1$-$C_2$-Alkyl) und Hydroxy), $C_2$-$C_6$-Alkenyl (das optional substituiert ist mit 1, 2 oder 3 Halogenatomen), $C_3$-$C_6$-Cycloalkyl (das optional substituiert ist mit 1, 2 oder 3 Halogenatomen und/oder mit 1 Substituenten, der ausgewählt ist aus $C_1$-$C_4$-Alkoxy und Hydroxy), worin eine C-C Bindung im Cycloalkylteil optional substituiert ist durch eine O-C, S-C oder C=C Bindung, $C_4$-$C_7$-Cycloalkylalkyl (das optional substituiert ist mit 1, 2 oder 3 Halogenatomen und/oder mit 1 Substituenten, der ausgewählt ist aus $C_1$-$C_4$-Alkoxy und Hydroxy), worin eine C-C Bindung im Cycloalkylteil optional substituiert ist mit einer O-C, S-C oder C=C Bindung, oder aus $CH_2Ar_2$, und
$Ar_1$ und $Ar_2$ jeweils unabhängig stehen für einen Phenylring oder einen 5- oder 6-gleidrigen Heteroarylring, wovon jeder optional substituiert ist mit 1, 2 oder 3 Substituenten (in Abhängigkeit von der Anzahl an verfügbaren Substitutionspositionen), die jeweils unabhängig ausgewählt sind aus $C_1$-$C_4$-Alkyl (das optional substituiert ist mit 1, 2 oder 3 Halogenatomen), $C_1$-$C_4$-Alkoxy (das optional substituiert ist mit 1, 2 oder 3 Halogenatomen), $C_1$-$C_4$-Alkylthio (das optional substituiert ist mit 1, 2 oder 3 Halogenatomen), -CO-O-($C_1$-$C_4$-Alkyl), Cyano, -NRR, -CONRR, Halogen und Hydroxy und/oder mit 1 Substituenten, der ausgewählt ist aus Pyridyl, Thiophenyl, Phenyl, Benzyl und Phenoxy, wovon jeder dieser Reste am Ring optional substituiert ist mit 1, 2 oder 3 Substituenten, die jeweils unabhängig ausgewählt sind aus Halogen, $C_1$-$C_4$-Alkyl (das optional substituiert ist mit 1, 2 oder 3 Halogenatomen), $C_1$-$C_4$-Alkoxy (das optional substituiert ist mit 1, 2 oder 3 Halogenatomen), Carboxy, Nitro, Hydroxy, Cyano, -NRR, -CONRR, $SO_2NRR$ und $SO_2R$), und
R jeweils unabhängig für H oder $C_1$-$C_4$-Alkyl steht,

oder ein pharmazeutisch akzeptables Salz hiervon.

**2.** Verwendung nach Anspruch 1, worin der selektive Wiederaufnahmeinhibitor von Norepinephrin eine Verbindung der Formel (IIIH) ist

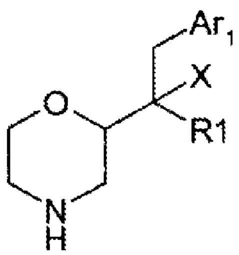

(IIIH)

worin

X, R1 und $Ar_1$ wie im Anspruch 1 definiert sind,

oder ein pharmazeutisch akzeptables Salz hiervon.

**3.** Verwendung nach Anspruch 1, worin der selektive Wiederaufnahmeinhibitor von Norepinephrin eine Verbindung der Formel (IVH) ist

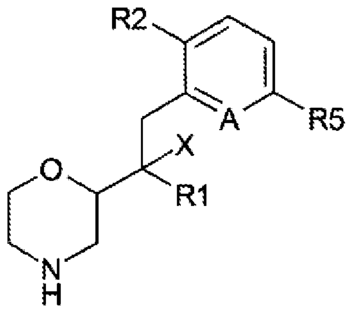

(IVH)

worin

X für OH oder $NH_2$ steht,
R1 steht für $C_1$-$C_6$-Alkyl (das optional substituiert ist mit 1, 2 oder 3 Halogenatomen und/oder mit 1 Substituenten, der ausgewählt ist aus $C_1$-$C_4$-Alkoxy (das optional substituiert ist mit 1, 2 oder 3 Fluoratomen), Cyano und Hydroxy), $C_3$-$C_6$-Cycloalkyl (das optional substituiert ist mit 1, 2 oder 3 Halogenatomen und/oder mit 1 Substituenten, der ausgewählt ist aus $C_1$-$C_4$-Alkoxy und Hydroxy), worin eine C-C Bindung im Cycloalkylteil optional substituiert ist durch eine O-C Bindung, oder $CH_2Ar_2$, worin $Ar_2$ ein Phenylring ist, der optional substituiert ist mit 1, 2 oder 3 Substituenten, die unabhängig ausgewählt sind aus $C_1$-$C_4$-Alkyl (das optional substituiert ist mit 1, 2 oder 3 Halogenatomen), $C_1$-$C_4$-Alkoxy (das optional substituiert ist mit 1, 2 oder 3 Halogenatomen), $C_1$-$C_4$-Alkylthio (das optional substituiert ist mit 1, 2 oder 3 Halogenatomen), Halogen und Hydroxy,
A für N oder CH steht,
R2 steht für $C_1$-$C_4$-Alkyl (das optional substituiert ist mit 1, 2 oder 3 Halogenatomen), $C_1$-$C_4$-Alkoxy (das optional substituiert ist mit 1, 2 oder 3 Halogenatomen), $C_1$-$C_4$-Alkylthio (das optional substituiert ist mit 1, 2 oder 3 Halogenatomen), Halogen, Hydroxy, Pyridyl, Thiophenyl, Phenyl (das optional substituiert ist mit 1, 2 oder 3 Halogenatomen, die ausgewählt sind aus Halogen, $C_1$-$C_4$-Alkyl (das optional substituiert ist mit 1, 2 oder 3 Halogenatomen) oder $C_1$-$C_4$-Alkoxy (das optional substituiert ist mit 1, 2 oder 3 Halogenatomen)) oder Phenoxy (das optional substituiert ist mit 1, 2 oder 3 Halogenatomen),
R5 steht für H, $C_1$-$C_4$-Alkyl (das optional substituiert ist mit 1, 2 oder 3 Halogenatomen), $C_1$-$C_4$-Alkoxy (das optional substituiert ist mit 1, 2 oder 3 Halogenatomen), $C_1$-$C_4$-Alkylthio (das optional substituiert ist mit 1, 2 oder 3 Halogenatomen), Halogen oder Hydroxy,

oder ein pharmazeutisch akzeptables Salz hiervon.

**4.** Verwendung nach Anspruch 1, worin der selektive Wiederaufnahmeinhibitor von Norepinephrin eine Verbindung der Formel (VH) ist

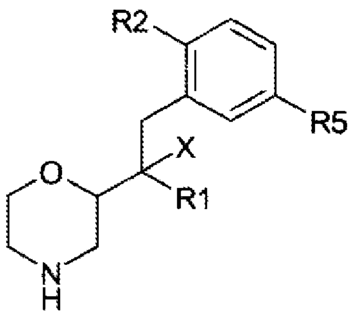

(VH)

worin

X für OH oder $NH_2$ steht,
R1 steht für $C_1$-$C_6$-Alkyl (das optional substituiert ist mit 1, 2 oder 3 Fluoratomen), $C_3$-$C_6$-Cycloalkyl, worin eine C-C Bindung im Cycloalkylteil optional substituiert ist durch eine O-C Bindung oder $CH_2Ar_2$, worin $Ar_2$ ein Phenylring ist, der optional substituiert ist mit 1 oder 2 Substituenten, die jeweils unabhängig ausgewählt sind aus $C_1$-$C_4$-Alkyl (das optional substituiert ist mit 1, 2 oder 3 Halogenatomen), $C_1$-$C_4$-Alkoxy (das optional substituiert ist mit 1, 2 oder 3 Halogenatomen), Halogen und Hydroxy,
R2 steht für $C_1$-$C_4$-Alkyl (das optional substituiert ist mit 1, 2 oder 3 Fluoratomen), $C_1$-$C_4$-Alkoxy (das optional substituiert ist mit 1, 2 oder 3 Fluoratomen) oder Phenyl (das optional substituiert ist mit 1, 2 oder 3 Fluoratomen), und
R5 für H oder F steht,

oder ein pharmazeutisch akzeptables Salz hiervon.

**5.** Verwendung nach Anspruch 1, worin der selektive Wiederaufnahmeinhibitor von Norepinephrin eine Verbindung der Formel (VIH) ist

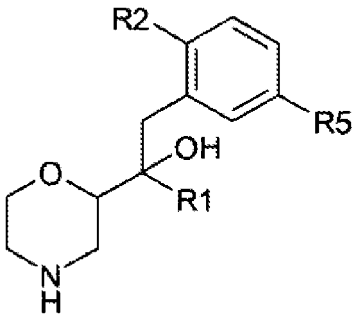

(VIH)

worin

R1 steht für $C_1$-$C_6$-Alkyl (das optional substituiert ist mit 1, 2 oder 3 Fluoratomen) oder $C_3$-$C_6$-Cycloalkyl, worin eine C-C Bindung im Cycloalkylteil optional substituiert ist durch eine O-C Bindung,
R2 steht für $C_1$-$C_4$-Alkyl (das optional substituiert ist mit 1, 2 oder 3 Fluoratomen), $C_1$-$C_4$-Alkoxy (das optional substituiert ist mit 1, 2 oder 3 Fluoratomen) oder Phenyl (das optional substituiert ist mit 1, 2 oder 3 Fluoratomen), und
R5 für H oder F steht,

oder ein pharmazeutisch akzeptables Salz hiervon.

**6.** Verwendung nach Anspruch 1, worin der selektive Wiederaufnahmeinhibitor von Norepinephrin eine Verbindung der folgenden Formel ist

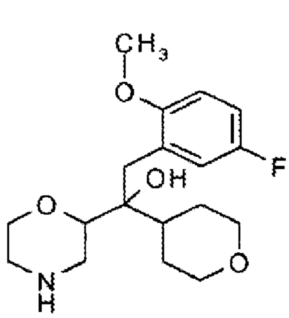

oder das Hydrochloridsalz hiervon.

## Revendications

1. Utilisation d'un inhibiteur sélectif du recaptage de la norépinéphrine pour la fabrication d'un médicament destiné au traitement de bouffées de chaleur ou de symptômes vasomoteurs, dans laquelle ledit inhibiteur sélectif du recaptage de la norépinéphrine est un composé de formule (IH) :

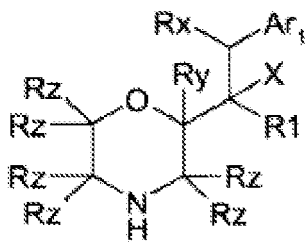

(IH)

dans laquelle,

X représente un groupe OH, alcoxy en C1-C4, $NH_2$ ou NH(alkyle en C1-C4) ;
Rx représente un atome d'hydrogène ou un groupe alkyle en C1-C4 ;
Ry représente un atome d'hydrogène ou un groupe alkyle en C1-C4 ;
chaque groupe Rz représente, de manière indépendante, un atome d'hydrogène ou un groupe alkyle en C1-C4, à la condition que pas plus de 3 groupes Rz puissent être un groupe alkyle en C1-C4 ;
R1 représente un groupe alkyle en C1-C6 (éventuellement substitué par 1, 2 ou 3 atome(s) d'halogène et/ou par un substituant choisi parmi un groupe alkylthio en C1-C4 (éventuellement substitué par 1, 2 ou 3 atome(s) de fluor), alcoxy en C1-C4 (éventuellement substitué par 1, 2 ou 3 atome(s) de fluor), cycloalcoxy en C3-C6, alkylsulfonyle en C1-C4, cyano, -CO-O(alkyle en C1-C2), -O-CO-(alkyle en C1-C2) et hydroxy) ; un groupe alcényle en C2-C6 (éventuellement substitué par 1, 2 ou 3 atome(s) d'halogène) ; un groupe cycloalkyle en C3-C6 (éventuellement substitué par 1, 2 ou 3 atome(s) d'halogène et/ou par 1 substituant choisi parmi un alcoxy en C1-C4 et un hydroxy) dans lequel une liaison C-C à l'intérieur de la fraction cycloalkyle est éventuellement remplacée par une liaison O-C, S -C ou C=C ; un groupe cycloalkylalkyle en C4-C7 (éventuellement substitué par 1, 2 ou 3 atome(s) d'halogène et/ou par un substituant choisi parmi un alcoxy en C1-C4 et un hydroxy) dans lequel une liaison C-C à l'intérieur de la fraction cycloalkyle est éventuellement remplacée par une liaison O-C, S-C, ou C=C ; ou $CH_2Ar_2$ ; et
Ar1 et Ar2 représentent chacun, de manière indépendante, un cycle phényle ou un cycle hétéroaryle de 5 ou 6 membres chacun de ceux-ci étant éventuellement substitué par 1, 2 ou 3 substituant(s) (en fonction du nombre de positions de substitution disponibles) choisis chacun de manière indépendante, parmi un groupe alkyle en C1-C4 (éventuellement substitué par 1, 2 ou 3 atome(s) d'halogène), alcoxy en C1-C4 (éventuellement substitué par 1, 2 ou 3 atome(s) d'halogène), alkylthio en C1-C4 (éventuellement substitué par 1, 2 ou 3 atome(s) d'halogène), -CO-O(alkyle en C1-C4), cyano, NRR, -CONRR, halogéno et hydroxy et/ou par 1 substituant choisi parmi un groupe pyridyle, thiophényle, phényle, benzyle et phénoxy chacun de ceux-ci étant éventuellement substitué sur le cycle par 1, 2 ou 3 substituant(s) choisi(s), chacun, de manière indépendante, parmi un atome d'halogène, un groupe alkyle en C1-C4 (éventuellement substitué par 1, 2, ou 3 atome(s) d'halogène), un groupe alcoxy en C1-C4 (éventuellement substitué par 1, 2, ou 3 atome(s) d'halogène), un groupe carboxy, nitro, hydroxy, cyano, -NRR, -CONRR, $SO_2NRR$ et $SO_2R$) ; et
chaque substituant R représente, de manière indépendante, un atome d'hydrogène ou un groupe alkyle en C1-C4 ;

ou bien un sel pharmaceutiquement acceptable de celui-ci.

2. Utilisation selon la revendication 1, dans laquelle ledit inhibiteur sélectif du recaptage de la norépinéphrine est un composé de formule (IIIH)

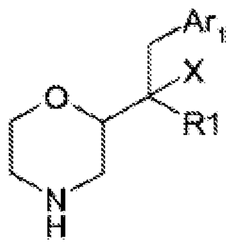

(IIIH)

dans laquelle, X, R1 et Ar1 sont tels que définis selon la revendication 1 ;
ou bien un sel pharmaceutiquement acceptable de celui-ci.

**3.** Utilisation selon la revendication 1, dans laquelle ledit inhibiteur sélectif du recaptage de la norépinéphrine est un composé de la formule (IVH)

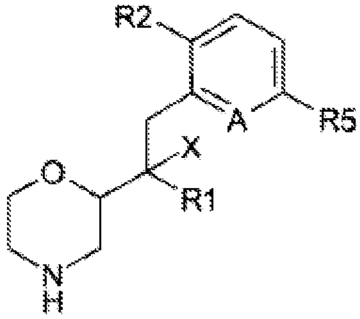

(IVH)

dans laquelle,

X représente un groupe OH ou $NH_2$ ;
R1 représente un groupe alkyle en C1-C6 (éventuellement substitué par 1, 2 ou 3 atome(s) d'halogène et/ou par 1 substituant choisi parmi un groupe alcoxy en C1-C4 (éventuellement substitué par 1, 2 ou 3 atome(s) de fluor), cyano, et hydroxy) ; un groupe cycloalkyle en C3-C6 (éventuellement substitué par 1, 2 ou 3 atome(s) d'halogène et/ou par un 1 substituant choisi parmi un alcoxy en C1-C4 et un hydroxy) dans lequel une liaison C-C à l'intérieur de la fraction cycloalkyle est éventuellement remplacée par une liaison O-C ; ou un groupe $CH_2Ar2$ dans lequel Ar2 représente un cycle phényle éventuellement substitué par 1, 2 ou 3 substituant(s) choisi(s) chacun, de manière indépendante, parmi un groupe alkyle en C1-C4 (éventuellement substitué par 1, 2 ou 3 atome(s) d'halogène), alcoxy en C1-C4 (éventuellement substitué par 1, 2 ou 3 atome(s) d'halogène), alkylthio en C1-C4 (éventuellement substitué par 1, 2 ou 3 atome(s) d'halogène), halogéno et hydroxy ;
A représente un atome d'azote ou CH ;
R2 représente un groupe alkyle en C1-C4 (éventuellement substitué par 1, 2 ou 3 atome(s) d'halogène), alcoxy en C1-C4 (éventuellement substitué par 1, 2 ou 3 atome(s) d'halogène), alkylthio en C1-C4 (éventuellement substitué par 1, 2 ou 3 atome(s) d'halogène), halogéno, hydroxy, pyridyle, thiophényle, phényle (éventuellement substitué par 1, 2 ou 3 substituant(s) choisi(s) chacun, de manière indépendante, parmi un atome d'halogène, un groupe alkyle en C1-C4 (éventuellement substitué par 1, 2 ou 3 atome(s) d'halogène), ou alcoxy en C1-C4 (éventuellement substitué par 1, 2 ou 3 atome(s) d'halogène)) ou phénoxy (éventuellement substitué par 1, 2 ou 3 atome(s) d'halogène) ;
R5 représente un atome d'hydrogène, un groupe alkyle en C1-C4 (éventuellement substitué par 1, 2 ou 3 atome (s) d'halogène), alcoxy en C1-C4 (éventuellement substitué par 1, 2 ou 3 atome(s) d'halogène), alkylthio en C1-C4 (éventuellement substitué par 1, 2 ou 3 atome(s) d'halogène), halogéno ou hydroxy ;

ou bien un sel pharmaceutiquement acceptable de celui-ci.

**4.** Utilisation selon la revendication 1, dans laquelle ledit inhibiteur sélectif du recaptage de la norépinéphrine est un composé de formule (VH)

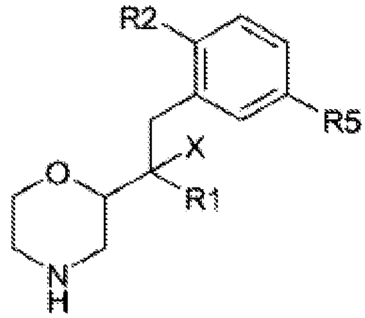

(VH)

dans laquelle,

X représente un groupe OH ou $NH_2$ ;
R1 représente un groupe alkyle en C1-C6 (éventuellement substitué par 1, 2 ou 3 atome(s) de fluor), un groupe cycloalkyle en C3-C6 dans lequel une liaison C-C à l'intérieur de la fraction cycloalkyle est éventuellement remplacée par une liaison O-C ou un groupe $CH_2$Ar2 dans lequel Ar2 représente un cycle phényle éventuellement substitué par 1 ou 2 substituant(s) choisi(s) chacun, de manière indépendante, parmi un groupe alkyle en C1-C4 (éventuellement substitué par 1, 2 ou 3 atome(s) d'halogène), alcoxy en C1-C4 (éventuellement substitué par 1, 2 ou 3 atome(s) d'halogène), halogéno et hydroxy ;
R2 représente un groupe alkyle en C1-C4 (éventuellement substitué par 1, 2 ou 3 atome(s) de fluor), alcoxy en C1-C4 (éventuellement substitué par 1, 2 ou 3 atome(s) de fluor) ou phényle (éventuellement substitué par 1, 2 ou 3 atome(s) de fluor) ;
et R5 représente un atome d'hydrogène ou de fluor ;

ou bien un sel pharmaceutiquement acceptable de celui-ci.

**5.** Utilisation selon la revendication 1, dans laquelle ledit inhibiteur sélectif du recaptage de la norépinéphrine est un composé de formule (VIH)

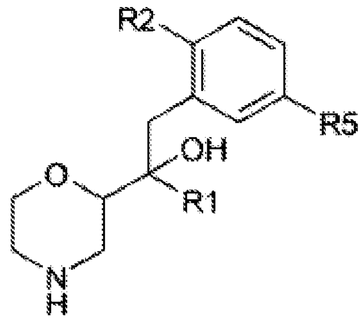

(VIH)

dans laquelle,

R1 représente un groupe alkyle en C1-C6 (éventuellement substitué par 1, 2 ou 3 atome(s) de fluor) ou cycloalkyle en C3-C6 dans lequel une liaison C-C à l'intérieur de la fraction cycloalkyle est éventuellement remplacée par une liaison O-C ;
R2 représente un groupe alkyle en C1-C4 (éventuellement substitué par 1, 2 ou 3 atome(s) de fluor), alcoxy en C1-C4 (éventuellement substitué par 1, 2 ou 3 atome(s) de fluor) ou phényle (éventuellement substitué par

1, 2 ou 3 atome(s) de fluor) ;
et R5 représente un atome d'hydrogène ou de fluor ;

ou bien un sel pharmaceutiquement acceptable de celui-ci.

**6.** Utilisation selon la revendication 1, dans laquelle ledit inhibiteur sélectif du recaptage de la norépinéphrine est un composé de formule

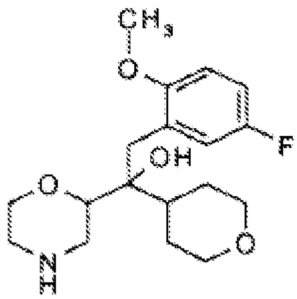

ou bien le sel chlorhydrate de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03037334 A
- MW 23533
- MW 24936
- MW 26338
- MW 26137
- MW 27338
- MW 28938
- MW 30131
- MW 31534
- MW 19123
- MW 26132
- MW 24734
- MW 24532
- MW 38753
- MW 29736
- MW 37347
- MW 31981
- MW 40945
- MW 31933
- MW 40155
- MW 34782
- MW 387
- MW 33383
- MW 36951
- MW 31584
- MW 38354
- MW 29336
- MW 42338
- MW 37982
- MW 41558
- MW 36191
- MW 37991
- MW 40153
- MW 34772
- MW 39756
- MW 30744
- MW 43751
- MW 38382
- MW 42961
- MW 33948
- MW 47362
- MW 37594
- MW 44760
- MW 39395
- MW 41354
- MW 35982
- MW 40957
- MW 35591
- MW 44952
- MW 35936
- MW 44162
- MW 38795
- MW 45961
- MW 40594
- MW 42954
- MW 37582
- MW 46552
- MW 41185


### Non-patent literature cited in the description


- *Psychosom. Med.,* 1965, vol. 27, 266
- *Med. Gynecol. Soc.,* 1969, vol. 4, 268
- *Br. J. Obstet. Gynaecol.,* 1977, vol. 84, 769
- *N. Engl. J. Med.,* 1981, vol. 305, 663
- *Urology,* 1980, vol. 16, 620
- *Arch. Intern. Med.,* 1991, vol. 151, 1842
- *Clin. Endocrinol. (Oxf,* 1985, vol. 22, 293
- *Can. J. Physiol. Pharmacol.,* 1987, vol. 65, 1312
- *Mayo. Clin. Proc.,* 2002, vol. 77, 1207
- *Ann. Intern. Med.,* 2000, vol. 132, 788
- *Br. Med. J.,* 1974, vol. i, 409
- *Med. J. Aust.,* 1986, vol. 144, 369
- *Fertil. Steril.,* 1985, vol. 43, 401
- *Br. J. Obstet. Gynaecol.,* 1981, vol. 88, 919
- *J. Clin. Endocrinol. Metab.,* 1984, vol. 58, 578
- *Clin. Endocrinol.,* 1985, vol. 22, 293
- *J. Clin. Oncol.,* 2002, vol. 20, 1583
- *JAMA,* 2003, vol. 289, 2827
- *Lancet,* 2000, vol. 356, 2059
- *N. Engl. J. Med.,* 1994, vol. 331, 347
- *Obstet. Gynecol.,* 1984, vol. 63, 1
- *Obstet. Gynecol.,* 1999, vol. 94, 225
- *Br. J. Obstet. Gynecol.,* 1998, vol. 105, 904
- *Neurology,* 2000, vol. 54, 2161
- *Obstet. Gynecol.,* 1998, vol. 72, 688
- *J. Clin. Oncol.,* 1998, vol. 16, 495
- *J. Clin. Oncol.,* 2001, vol. 19, 2739
- *J. Nutr.,* 2001, vol. 131 (11), 3095s
- **WONG et al.** *Drug Development Research,* 1985, vol. 6, 397

- Protective Groups in Organic Synthesis. Theodora W. Greene and Peter G.M. Wuts, John Wiley & Sons, Inc, 1999, 494-653
- **CHARETTE, A.B. ; GAGNON, A ; JANES, M ; MELLON, C.** *Tetrahedron Lett,* 1998, vol. 39 (29), 5147-5150
- **CHARETTE, A.B. ; GAGNON, A.** *Tetrahedron: Asymmetry,* 1999, vol. 10 (10), 1961-1968
- **FIESER, L.F. ; FIESER, M.F.** Reagents for Organic Synthesis. John Wiley and Sons Inc, vol. 1, 415-424
- **MARCH, J.** Advanced Organic Chemistry. John Wiley and Sons Inc, 558-561
- *JACS,* 2000, vol. 122, 4020-4028
- *Molecular Brain Research,* 1992, vol. 15, 161-166
- *Nature,* 1991, vol. 350, 350-354
- *Proceedings of the National Academy of Sciences of the USA,* 1993, vol. 90, 2542-2546
- **J. GOBEL ; D.L. SAUSSY ; A. GOETZ.** *J. Pharmacol. Toxicol.,* 1999, vol. 42, 237-244
- **RAMAMOORTHY, S. ; GIOVANETTI, E. ; QIAN, Y. ; BLAKELY, R.** *J. Biol. Chem.,* 1998, vol. 273, 2458
- **MOODY GC ; GRIFFIN SJ ; MATHER AN ; MCGINNITY DF ; RILEY RJ.** Fully automated analysis of activities catalyzed by the major human liver cytochrome P450 (CYP) enzymes: assessment of human CYP inhibition potential. *Xenobiotica,* 1999, vol. 29 (1), 53-75
- **SIMPKINS et al.** *Life Sci.,* 1983, vol. 32, 1957-1966
- **MERCHENTHALER et al.** *Maturitas,* 1998, vol. 30, 307-316
- **GEHLERT et al.** *Life Sci.,* 1995, vol. 56 (22), 1915-1920